# EUROPEAN PATENT APPLICATION

(11) **EP 2 546 352 A1**
(43) Date of publication of application: **16.01.2013**
(21) Application number: 11174126.0
(22) Date of filing: 15.07.2011
(51) Int. Cl.: C12P 7/64

(54) **Process for producing lipids from palm oil production residues**

(71) Applicant: Neste Oil Oyj, 02150 Espoo (FI)
(72) Inventor: Koskinen, Perttu, FI-00820 Helsinki (FI); Lakaniemi, Aino-Maija, FI-33720 Tampere (FI); Marjakangas, Jatta, FI-33720 Tampere (FI); Puhakka, Jaakko, FI-33230 Tampere (FI)
(74) Representative: Knuth-Lehtola, Sisko Hillevi

(57) **Abstract**

The present invention relates to a process for treating palm oil production residues. The process comprises one or more aerobic treatment steps, wherein lipid producing heterotrophic and/or mixotrophic microorganisms are cultivated in a medium comprising palm oil production residues as carbon and nutrient source(s) under aerobic cultivation conditions. The solids fraction is separated from the liquid fraction; and lipids are recovered from the solids fraction. The treatment steps can be used in combination with anaerobic or other aerobic treatment steps. By treating the POME according to this invention the BOD content of POME can be efficiently decreased in aerobic waste water treatment and thereby also the potential methane emissions created in anaerobic treatment can be reduced.

## Description

The present invention relates to a process for treating palm oil production residues, such as palm oil mill effluent and lignocellulosic palm oil production residues, such as oil palm empty fruit bunches. The invention relates in particular to the use of palm oil production residues as carbon and/or nutrient source for lipid producing microorganisms and to the use of the produced lipids as biofuel or lubricant or as a starting material for biofuel or lubricant.

### Background of the invention

Palm oil (including palm kernel oil) is a vegetable oil which is nowadays produced in highest amounts. The annual production was about 48 Million tons in 2008. The main uses of palm oil today include food use (80%), oleochemicals (15%) and biofuels (5%). Production of palm oil results in high quantities of residues, including liquid residues, such as palm oil mill effluent (POME), and lignocellulosic residues, such as oil palm empty fruit bunch (EFB), palm fibre and palm kernel shell. The palm fibre and kernel shell are typically combusted for energy production within the mill. The other solid residue, EFB (accounting about 20% of fresh fruit bunch) is typically not combusted due to its high moisture content (65%), but it can be composted and used as a mulch. The production of palm oil is typically performed by wet milling, which requires high amount of water and steam for washing and sterilization steps. The wet milling process generates high volumes of waste water called palm oil mill effluent, POME (about 1 to 4 t of POME per t crude of palm oil). POME is a colloidal suspension typically containing 94-96% water, 0.5-2.0 % oil and 4-5% total solids including 2-4% suspended solids originating from the mixture of a sterilizer condensate, separator sludge and hydrocyclone waste water. POME is a high strength waste water, with biological oxygen demand (BOD) typically from 25 to 60 g/L and chemical oxygen demand (COD) of 30 to 100 g/L, which need to be treated before discharged into watercourses. Presently, the treatment of POME occurs primeraly in anaerobic lagoons. In anaerobic lagoons it forms substantial amounts of methane (due to the high organic load in POME). The methane, if not collected, results in significant greenhouse gases (GHG) emissions. Typical sizes of palm oil mills range from 100 kt to 500 kt of fresh fruit bunch per year, corresponding approximately the production of 20 million liter to 500 million liter of POME per plant per year.

Residual oil is one of the key ingredients of POME which influences the high chemical oxygen demand (COD) and biological oxygen demand (BOD) values. The maximum allowable limit set by the Malaysian Department of Environment (DOE) for residual oil is 50 mg/l. and for BOD 100 mg/l The oil droplets occur in two phases. They are bound in the solids, such as plant matrix, of POME and also suspended in the supernatant of POME.

Several suggestions have been made to utilize palm oil mill residues instead of combustion or composting or releasing it into the environment. The production of several high value products from POME has been suggested, such as antibiotics, bioinsecticides, polyhydroxyalkanoates, citric acid and enzymes (Wu et al. 2009). Also the production of biofuels, such as acetone-butanol-ethanol from POME has been studied (Wu et al. 2009). In addition several studies exist on production of hydrogen during treatment of POME (Wu et al. 2009). Algae have been used in the tertiary treatment of POME in palm oil mills and the oil production by algae has been also suggested (Lam and Lee 2011). Further the treatment of POME by hydrocarbon-degrading yeasts, such as *Yarrowia lipolytica,* has been proposed (Oswal et al. 2002). Enzyme production by fungi from palm oil production residues has been suggested, such as cellulase, xylanase, peroxidise or lipase production from POME (Wu et al. 2009) or cellulase production from oil palm EFB. Palm oil has been used as a carbon source in cultivation of streptomycetes for production of bioactive metabolites and some chemicals to organic chemical industry, e.g. lipases (Vishnupriya et al. 2010).

Although several suggestions and attempts have been made for better utilization of palm oil production residues, the residues still form a significant waste and enrichment challenge for the environment.

### Summary

One object of the present invention is to provide a process for treating palm oil production residues. More specifically one object of the invention is to provide a process for treating palm oil mill effluent alone or together with other palm oil production residues.

Another object of the invention is to provide a process for utilizing palm oil production residues in such a way that the amount of organic carbon and nutrients released into the environment are reduced.

Yet another object of the invention is to provide a process to valorize the palm oil production residues during their treatment

These objects are achieved by the present invention characterized by the features that are enlisted in the independent claims. Dependent claims represent the preferred embodiments of the invention.

The present invention provides a process for treating palm oil production residues by using lipid producing microorganisms i.e. by producing and recovering oil produced by microorganisms. In the present invention it has surprisingly been found that lipid producing microorganisms accumulate high amounts of lipids and in particular the triacylglycerols (TAGs) when cultivated on waste and/or residue materials derived from palm oil production as carbon and/or nutrient source.

The present invention thus provides a process for treating palm oil production residues which process utilizes carbon and/or nutrients contained in palm oil production residues. The process preferably uses effluent from palm oil production process optionally added with lignocellulosic palm oil production residues as cultivation medium for lipid accumulating microorganisms.

The present invention provides in particular a process for treating effluent from palm oil production process i.e. reduction of organic load in the effluent. This process can be combined with the oil production and/or recovery of oil by microorganisms.

The process results in efficient accumulation of lipids in microorganisms and the present invention thus provides also a new raw material source for microbial lipid production.

The process also results in the recovery of existing lipids in the palm oil production residues that are not easily recoverable by traditional methods.

The process results in reduction in the amount of organic carbon and nutrients released into the environment.

Furthermore, the present invention provides a new process for lipid production for biofuel and lubricant use.

In one aspect the present invention provides a process for treating palm oil production residue(s), which comprises one or more aerobic treatment steps, wherein lipid producing heterotrophic and/or mixotrophic microorganisms are cultivated in a medium comprising palm oil production residue(s) as carbon and/or nutrient source(s) under aerobic cultivation conditions; the solids fraction is separated from the liquid fraction; and lipids are recovered from the solids fraction. Preferably the cultivation process is an aerated cultivation process.

The palm oil production residue(s) comprise(s) palm oil mill effluent (POME), and optionally lignocellulosic palm oil production residues, such as oil palm empty fruit bunch, palm fruit fibre (mesocarp fibre), palm kernel shell, palm kernel cake, oil palm wood, or oil palm fronds, POME solids or fractions thereof.

In one embodiment the process uses palm oil mill effluent (POME) and optionally lignocellulosic residues from palm oil production, such as oil palm empty fruit bunch, palm fruit fibre (mesocarp fibre), palm kernel shell, palm kernel cake, POME solids or fractions thereof, oil palm wood, or oil palm fronds or hydrolysates thereof, preferably oil palm empty fruit bunches (EFB), POME solids or fractions thereof, palm fruit fibre, palm kernel shell or palm kernel cake, or hydrolysates thereof, more preferably oil palm empty fruit bunches (EFB), palm fruit fibre (mesocarp fibre), POME solids or fractions thereof, palm kernel shell, or hydrolysates thereof.

As such POME may comprise varying amounts of POME solids, which is typically 4-5% of POME weight. In one embodiment of the invention lignocellulosic palm oil production residue(s) is/are added to the cultivation medium in an amount of 0.1 - 400 g as dry weight per liter cultivation medium, preferably 1 - 300 g/l, more preferably 2 to 200 g/l.

In another embodiment the process uses a cultivation medium which comprises an additional carbon source, such as purified sugar(s) or crude sugar(s) or any mixtures thereof. The amount of purified or crude sugar(s) is 0.1 - 200 g per liter cultivation medium, preferably 1 -150 g/l, more preferably 2 - 100 g/l.

In one further embodiment the cultivation medium comprises also cell waste or residue (cell debris). The amount of cell waste or residue is 1-300 g per liter cultivation medium, preferably 2 - 200 g per liter cultivation medium, more preferably 5 - 200 g/l, in certain embodiments 5 - 20 g/l.

In one further embodiment the cultivation medium comprises sugars from lignocellulosic materials, such as lignocellulosic hydrolysates, from sources other than palm oil production residues.

In certain embodiments POME is concentrated before use in the cultivations until the dry weight of POME is 10 - 80 wt-%.

In one embodiment of the invention, solid fractions in POME are separated and these or fractions or hydrolysates thereof are used in the cultivation medium for lipid production with microorganisms.

The process for treating palm oil production residue(s) comprises at least one aerobic process or one or more aerobic processes, which use heterotrophic and/or mixotrophic lipid producing microorganisms. The process comprises alternatively or in addition one or more aerobic processes, which use autotrophic and/or mixotrophic lipid producing micro-organisms.

In one preferred embodiment of the invention the process comprises at least one aerobic and aerated process, which uses heterotrophic and/or mixotrophic lipid producing micro-organisms. In one preferred embodiment of the invention the process comprises at least one aerobic and aerated process, which uses heterotrophic lipid producing microorganisms.

The present invention provides in particular a process for production of lipids by using heterotrophic microorganisms. The heterotrophic microorganisms comprise yeasts, fungi, in particular moulds, bacteria or heterotrophic microalgae or any mixed population of them. Preferably the heterotrophic microorganisms comprise yeasts, fungi, in particular moulds, or bacteria or any mixed population of them. The origin of the mixed population is in one embodiment of the invention palm oil mill effluent (POME).

In certain embodiments the microorganisms are a mixed culture of lipid producing and non-lipid producing microorganisms, preferably such mixed culture where lipid producing microorganisms are enriched.

In certain embodiments the microorganisms are a mixed culture of heterotrophic, mixotrophic and autotrophic lipid producing microorganisms, preferably of heterotrophic and mixotrophic lipid producing microorganisms.

In a preferred embodiment the microorganisms comprise *Actinomycetes,* in particular *Streptomyces,* more preferably *Streptomyces roseosporus, Streptomyces albus,* Streptomyces griseus, *Streptomyces peucetius, Streptomyces aureofaciens Streptomyces lividans, Streptomyces coelicolor, Streptomyces hygroscopicus, Streptomyces avermitilis, Streptomyces milbemycinius* or *Streptomyces lydicus.* Most preferably the microorganisms comprise species selected from the group of *Streptomyces roseosporus, Streptomyces albus* and *Streptomyces milbemycinius.*

In one preferred embodiment of the invention the microorganisms comprise fungi, in particular lipid producing filamentous fungi or yeast.

In one further aspect the lipids produced according to this invention are used as biofuel, as a component of biofuel or as a starting material for biofuel production. The biofuel is preferably biodiesel or renewable diesel, gasoline and/or jet fuel.

In one further aspect the lipids produced according to this invention are used as lubricant or as a starting material for lubricant production.

In one embodiment of the invention the process comprises one or more anaerobic treatment steps, from which the effluent is introduced to aerobic and preferably aerated heterotrophic and/or mixotrophic cultivation with lipid accumulating microorganisms.

In one further embodiment of the invention the process comprises one or more aerobic and preferably aerated treatment steps with heterotrophic and/or mixotrophic organisms from which the effluent is introduced to anaerobic treatment.

In one yet further embodiment of the invention the process comprises one or more anaerobic treatment steps, from which the effluent is introduced to aerobic auto- and/or mixotrophic cultivation with lipid accumulating microorganisms.

The anaerobic process typically comprises an anaerobic digestion process, a hydrogen production process or an acidogenesis process or more than one of these processes.

In one yet further embodiment of the invention the effluent from heterotrophic and/or mixotrophic aerobic, preferably aerated cultivation with lipid accumulating microorganisms is introduced into auto- and/or mixotrophic lipid production with for example microalgae and/or cyanobacteria.

The separated water (effluent) from lipid production may be recirculated to the palm oil production process, used in irrigation, introduced to anaerobic digestor, introduced to algae pond, introduced to aerobic waste water treatment or released to the environment optionally after further treatment steps.

The invention results in many significant advantages. One advantage of the invention is that by treating the POME according to this invention the BOD content of POME can be efficiently decreased in aerobic waste water treatment and thereby also the potential methane emissions created in anaerobic treatment can be reduced. A remarkable advantage of the invention is that significant amounts of oil can be recovered and produced from palm oil production residues, such as EFB, palm fibre, palm kernel shell and POME. The conversion of palm oil production residues to oil leads to increase of oil production capacity in palm oil mills. Further advantage of the invention is the valorization of palm oil production residues and conversion of residues to higher value products such as oil.

### Brief description of the drawings

Figure 1 presents trends of TAG accumulation in medium containing POME, EFB and cell waste in repeated cultivations.
Figure 2 presents soluble chemical oxygen demand (CODₛ) removals during treatment of POME with mixed culture as averages of two replicate cultures. Standard deviation was as by the error bars.
Figure 3 shows total chemical oxygen demand (CODₜₒₜ) results during treatment of POME with mixed cultures in the beginning (start) of treatment and after 14 d (end) of treatment.
Figure 4 presents. lipid concentration in cultivation medium and lipid content in biomass after 14d treatment of POME with mixed culture.
Figure 5 presents dissolved chemical oxygen demand during treatment of POME with carbon source (glucose) additions. The arrows indicate the occurrence of 2g/l glucose additions.
Figure 6 shows lipid concentration in cultivation medium and lipid content in biomass after 7 days treatment of POME with additions of carbon source in cultivation with mixed culture.
Figure 7 shows soluble chemical oxygen demand (CODₛ) removal during treatment of POME with mixed cultures with and without additions of sugar mixture.
Figure 8 presents lipid content as mass percentage from biomass dry weight on cultivation days 0 and 8 during treatment of POME with and without addition of sugar mixture.
Figure 9 shows microscope images of the mixed culture from treatment of POME with addition of sugar mixture taken on cultivation days 0 (left) and 5 (right).
Figure 10 presents lipid content as mass percenrage from biomass dry weight on cultivation days 0 and 4 during treatment of POME with and without addition of cellulose.
Figure 11. Microorganisms of POME with phase contrast microscopy after 0, 1, 2 and 3 days incubation.
Figures 12 - 19 present process schemes I to VII

### Detailed description of the invention

The term "lipid" refers to a fatty substance, whose molecule generally contains, as a part, an aliphatic hydrocarbon chain, which dissolves in nonpolar organic solvents but is poorly soluble in water. Lipids are an essential group of large molecules in living cells. Lipids are, for example, fats, oils, waxes, wax esters, sterols, terpenoids, isoprenoids, carotenoids, polyhydroxyalkanoates, fatty acids, fatty alcohols, fatty acid esters, phospholipids, glycolipids, sphingolipids and acylglycerols. The term "lipid" and "oil" are used in this description synonymously.

The term "acyglycerol "refers to an ester of glycerol and fatty acids. Acylglycerols occur naturally as fats and fatty oils. Examples of acylglycerols include triacylglycerols (TAGs, triglycerides), diacylglycerols (diglycerides) and monoacylglycerols (monoglycerides).

By the term "palm oil production residues" is meant fractions containing biomaterial formed in the palm oil production, especially in palm oil milling process. Such fractions include, but are not limited to palm oil mill effluent or fractions thereof, to lignocellulosic palm oil production residue(s), such as oil palm empty fruit bunch, palm fruit fibre (mesocarp fibre), palm kernel shell, palm kernel cake, oil palm wood (e.g. trunks, branches, roots) or fractions thereof, or oil palm fronds (leaves) or POME solids.

By the term "palm oil mill effluent" or "POME" is meant an aqueous side stream generated in palm oil milling process. The POME can include one or more aqueous fractions generated in milling process, such as sterilizer condensate (sterilizer effluent), separator sludge (separator or centrifuge effluent) and/or hydrocyclone waste water (hydrocyclone effluent).

By the term "palm oil mill effluent solids" or "POME solids" is meant solid fraction in palm oil mill effluent. This typically contains residues of lignocellulosic material or their fractions and residues released from oil palm plant matrix, such as from oil palm fruit and empty fruit bunch in palm oil production process. The palm oil mill effluent solids can be separated from POME with several methods, such as by filtration.

By the term oil palm "empty fruit bunch" or "EFB" is meant a lignocellulosic residue material that is generated when oil is removed from oil palm fruit bunches. In addition to lignocellulosics, EFB can contain oil residues.

At present, EFB is composted to provide mulch as soil improver material or combusted in boilers to provide energy for palm oil production.

By the term "lignocellulosic palm oil production residues" is meant residues derived from palm oil production that include lignocellulose or fractions thereof, such as oil palm empty fruit bunch, palm fruit fibre (mesocarp fibre), palm kernel shell, palm kernel cake, oil palm wood (e.g.. trunks, branches, roots) or fractions thereof, or oil palm fronds (leaves) or lignocellulose fraction in POME, i.e. POME solids.

By heterotrophic oil accumulation or oil production is meant here oil accumulation or oil production by microorganisms that are capable of converting organic molecules to lipids, especially storage lipids. Such organisms typically possess ATP:citrate lyase enzyme activity through which they are capable of storing lipids in their cells.

By autotrophic oil accumulation or oil production is meant here oil accumulation or oil production by microorganisms that are able to do photosynthesis and to produce lipids, and thus able to convert CO₂ to lipids, especially storage lipids.

By mixotrophic oil production is meant here oil accumulation or oil production by microorganisms that are able to do photosynthesis and thus able to convert CO₂ to lipids, and in addition able to convert organic molecules to lipids, especially storage lipids.

By "mixed culture process" is meant here a microbial cultivation process that includes two or more microbial strains, i.e. uses mixed microbial cultures or aims at maintaining two or more microbial strains in cultivation process. Mixed microbial cultures can be made artificially by introducing two or more strains to cultivation system or mixed microbial cultures can be derived and/or enriched from nature or from industrial processes.

In various embodiment of the invention palm oil production residues are used as a supplement or as the main carbon and/or nutrient source, preferably as the main carbon and/or nutrient source in the medium, in which microorganisms are cultivated.

In the cultivations according to this invention is used a cultivation medium (or broth) which comprises palm oil mill effluent and optionally other palm oil production residues. In certain embodiments of the invention the cultivation medium comprises POME and lignocellulosic palm oil production residues, such as EFB (Empty fruit bunch) or palm fibre or palm kernel shell or POME solids. In certain embodiments the cultivation medium comprises POME and microbial cell waste or sludge from waste water treatment and optionally EFB or other lignocellulosic palm oil production residue. POME, lignocellulosic palm oil production residues and microbial cell waste or sludge can be used alone or as various combinations. POME and lignocellulosic palm oil production residues can be used as a supplemental or main carbon and/or nutrient source, in certain embodiments as the sole or main carbon and/or nutrient source. POME typically contains oil remains that are not separated in processing steps before waste water treatment.

Cell waste may be any microbial cell residue or debris obtained after cultivation of microbial cells and isolation of any desired product from the culture broth. Cell waste can be also sludge formed in the microbiological waste water treatment. Sludge mainly comprises microbial biomass, and poorly biodegradable and non-biodegradable material. Preferably, the microbial cell residue is a spent culture, which means biomass obtained after recovery of lipids from the microorganism cells or sludge by any method, e.g. by extraction. Microbial cell residue can be also excess sludge formed in the aerobic waste water treatment or sludge formed in anaerobic waste water treatment, such as in anaerobic digestion.

By "aerated treatment" or "aerated cultivation" or "aerated and aerobic treatment" is meant microbiological treatment of waste waters and/or wastes, where medium, for example gas, containing oxygen, such as air or oxygen enriched air, is introduced to the process (i.e. to the cultivation) by aeration, in particular by mechanical aeration. The aeration comprises for example blasting, purging or bubbling a medium containing oxygen, such as air, oxygen, or compressed air, through diffusers or spargers or the like. The aeration provides oxygen to aerobic, heterotrophic and/or mixotrophic microorganisms.

Autotrophic and/or mixotrophic microorganisms are capable of producing oxygen and therefore, if/since oxygen is produced the cultivation conditions maintain aerobic typically without aeration.

By "aerobic treatment" or "aerobic cultivation" or "aerobic conditions" is meant a microbiological treatment, cultivation or conditions, where dissolved oxygen concentration in the liquid is at least 0.001 mg/l, i.e. above 0 mg/l.

The aeration is preferably arranged in certain embodiments of the invention that way that the dissolved oxygen concentration is at least 0.1 mg/l, preferably at least 0.5 mg/l, more preferably at least 1 mg/l, still more preferably at least 2 mg/l, even more preferably at least 5 mg/l. For example in aerobic, preferably aerated ponds dissolved oxygen concentration in the liquid is at least 0.001 mg/l, i.e. above 0 mg/l, preferably at least 0.5 mg/l, more preferably at least 2 mg/l, even more preferably at least 5 mg/l.

Preliminary treatment of waste water include process step(s), where oil and/or suspended material is removed from waste water mainly by non-microbiological methods, such as screening, filtration, including e.g. sand filtration, membrane filtration, ultrafiltration etc., settling, flotation, skimming of oil etc

By "primary treatment of waste water" is meant microbiological treatment unit operation(s) with which the highest decrease of the organic content, such as BOD or COD, of waste water is obtained compared to other microbiological unit operations in a certain waste water treatment process.

The present invention comprises the utilization of palm oil production residues, especially palm oil mill effluent (POME) and lignocellulosic palm oil production residues, such as empty fruit bunches (EFB), palm fibre, POME solids or palm kernel shells, for the production of oil by heterotrophic and/or mixotrophic microorganisms, such as yeasts, moulds, bacteria or algae or mixed cultures thereof. The process can be utilized in the treatment of these residues, especially POME, to decrease their environmental impacts, for example methane production while simultaneously recovering oil that can be utilized e.g. as biofuel production feedstock. Certain microorganisms, such as yeasts, moulds, bacteria and heterotrophic algae utilize the oil and sugars and other biodegradable components in palm oil production residues to accumulate oil in microbial cells; such microorganisms are often called as oleaginous microorganisms. The micro-organisms can be harvested from cultivation medium, oil can be recovered from microbial cells, and used as feedstock for biofuel, such as e.g. biodiesel (FAME) or renewable diesel or jet-fuel, or lubricant production. The residual biomass from lipid production, after oil recovery from microorganisms, can be used for energy production, e.g. combustion and anaerobic digester, as animal feed or reused in the cultivation medium and the residues, such as ash or sludge from these processes can be used as fertilizer.

According to the present invention the sugars in lignocellulosic palm oil production residues are utilized for production of oil by microorganisms, while the remaining fraction (i.e. lignin) can be utilized for energy production. The oil-accumulating microorganisms are capable of producing lipids directly (without hydrolysis) from lignocellulosic palm oil production residues or alternatively they can produce lipids from pre-treated, e.g. hydrolysed lignocellulosic palm oil production residues.

According to the present invention POME is used as raw material for production of oil by microorganisms. The processes of the invention decreases the organic load of the waste water while simultaneously producing and accumulating oil by microorganisms. Oil can be used e.g. as biofuel or as feedstock for biofuel production. The treatment of POME under aerated and/or aerobic treatment(s) by microorganisms significantly reduces the organic load of POME. The organic load as COD, in particular as soluble COD, is decreased at least 10%, more preferably at least 20%, more preferably at least 30%, more preferably at least 40%, more preferably at least 50%, more preferably at least 60%, more preferably at least 70%, more preferably at least 80%, even more preferably at least 90% compared to the initial COD content in POME, i.e. the COD content in fresh waste water from the palm oil procuction process.

The water from oil production process can go further to anaerobic digestion, e.g. biogas production, if not adequately purified. Typically, oil is considered as a difficult compound in anaerobic digestion, or ethanol or butanol production which have been proposed to POME, but was found to be very suitable compound in lipid accumulation process by microorganisms. POME and lignocellulosic palm oil production residues, such as EFB, contain palm oil lipid residues which can be directly taken up by lipid accumulating microorganisms and included as lipid inclusions in microbial cells. Thus, it was surprisingly found that certain microorganisms are able to recover residual oil from palm oil production residues. In POME, the oil is suspended to the liquid phase and part of the oil is bound into the plant residues remaining in POME, i.e. plant matrix. The oil bound to the plant residues cannot be efficiently separated by physical methods used in palm oil mills, such as centrifugation or settling. The microbiologial treatment according to this invention provides a method to remove and recover oil remaining in POME. The oil accumulating microorganisms can uptake the oil included in POME, including oil bound to the plant residues, and incorporate the oil in lipid inclusions inside the cells. Microorganisms can be collected and oil can be recovered from micro-organism biomass, e.g. by chemical extraction. Also lignocellulosic residues from palm oil production, such as EFB or POME solids include oil. Microorganisms can also be used to recover oil from these residues.

Furthermore, it was surprisingly also found that addition of lignocellulosic palm oil production residues, such as palm oil empty fruit bunches, to microbial cultures growing in POME, increased the lipid production by microorganisms. Microorganisms are able to convert the organic compounds in palm oil production residues, such as carbohydrates, into lipids.

According to one embodiment of the invention, lipid production process from POME, optinally with other palm oil production residue(s) can be integrated as a part of POME waste water treatment process. Aerobic treatment of POME including lipid accumulation by microorganisms is preferably installed as a primary method for treatment of POME, which mainly decreases the organic content, such as BOD or COD in POME. The aerobic treatment including oil accumulation can be followed by anaerobic treatment, or another aerobic treatment e.g. including algae, such as mixotrophic algae including oil accumulation. The microorganism cells or sludge from aerobic treatment with lipid accumulation is collected. The lipids can be recovered from the lipid-rich microbial cells or sludge by e.g. chemical extraction. The aerobic process described herein significantly reduces the organic content in POME and does not produce methane, which is a powerful green house gas. Thus, this process can potentially enable lipid production from POME and optionally from other palm oil production residues, and in addition waste water treatment to reduce environmental load in POME and improve green house gas (GHG) balance of palm oil production process. The residual biomass after oil recovery is suitable for anaerobic digestion, preferably with methane capture. Anaerobic sludge can be used as a fertilizer. Alternatively, the residual biomass is composted to provide mulch that can be used as a fertilizer. Alternatively, anaerobic sludge is fed to a lipid accumulation process.

The aerobic process for the treatment of POME optinally with other palm oil production residue(s) can rely on the use of single strain of microorganisms, or mixed culture of mi-croorgaisms, preferably such mixed culture where lipid accumulating microorganisms are enriched.

According to one embodiment of the invention, anaerobic treatment, such as anaerobic digestion, preferably with methane capture, is first performed to POME optinally with other palm oil production residue(s). From the anaerobic treatment the effluent and possibly sludge is led to aerobic treatment with lipid accumulating organisms, such as hetero and/or mixotrophic oil accumulating microorganisms. The lipid-rich microorganism cells or sludge from aerobic treatment with lipid accumulation is collected, and oil is recovered from the cells or sludge by e.g. chemical extraction.

According to one embodiment of the invention, the sludge from anaerobic treatment of POME, such as anaerobic digestion of POME, is fed to a aerobic lipid production process by microorganisms from palm oil production residues, such as lignocellulosic palm oil production residues. The anaerobic sludge serves as nutrient and/or carbon source for heterorophic and/or mixoptrophic lipid production organisms.

According to one embodiment of the invention, the POME optinally with other palm oil production residue(s) is treated by first leading waste water to anaerobic reactor for acidogenesis, i.e. production of short chain organic acids and alcohols, and hydrogen by microorganisms. The effluent and possibly sludge from acidogenesis reactor containing organic acids and alcohols is led to treatment with lipid accumulating organisms, such as hetero and/or mixotrophic oil accumulating microorganisms. The lipid accumulating micro-organisms convert the organic acids and alcohols to lipids. The lipid-rich microorganism cells or sludge from aerobic treatment with lipid accumulation is collected, and oil is recovered from the cells or sludge by e.g. chemical extraction.

The process for treating palm oil production residue(s) according to the present invention comprises one or more of the following steps:
(1) Preliminary treatment
(2) Aerobic, preferably aerated treatment with hetero/mixotrophic microorganisms
(3) Aerobic treatment with auto/mixotrophic microorganisms
(4) Oil recovery
(5) 2nd aerated treatment with hetero/mixotrophic organisms using excess sludge from 1st aerated treatment
(6) Anaerobic treatment using anaerobic digestion and/or acidogenesis process.

The process according to the present invention comprises preferably at least steps (2) or (3), more preferably at least step (2), or both steps (2) and (3), and step (4). The process comprises optionally step (1).

Various embodiments of the invention has been described in the following process schemes I to VII.

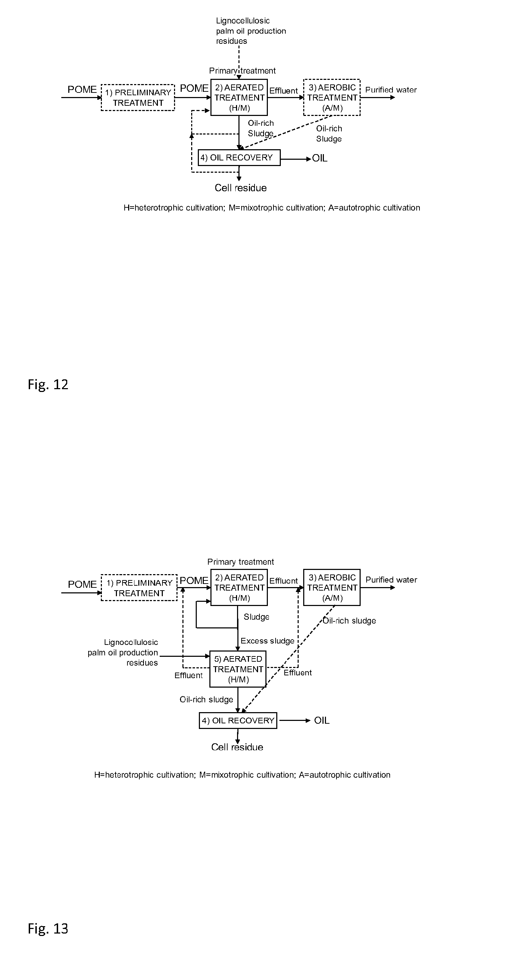

Scheme I presents the treatment of POME with hetero/mixotrophic process (2) including microbial oil accumulation combined optionally with auto/mixotrophic process (3) with oil accumulation. Aerobic, preferably aerated treatment with microbial oil accumulation may be supplemented with lignocellulosic palm oil production residues or hydrolysates thereof. Oil recovery (4) is performed to oil-rich sludge from aerated and optionally from aerobic treatment. A preferred embodiment is presented in Figure 12.

Scheme II presents the treatment of POME with aerobic, preferably aerated treatment using hetero/mixotrophic microorganisms combined with treatment using auto/mixotrophic organisms. Excess sludge from aerobic aerated treatment (2) is fed to second aerobic aerated process (5), which uses hetero/mixotrophic oil accumulating microorganisms. Second aerobic aerated treatment process is fed with lignocellulosic palm oil production residues or hydrolysates thereof to increase lipid production. Oil recovery (4) is performed to oil-rich sludge from aerated treatment and optionally from aerobic treatment. A preferred embodiment is presented in Figure 13.

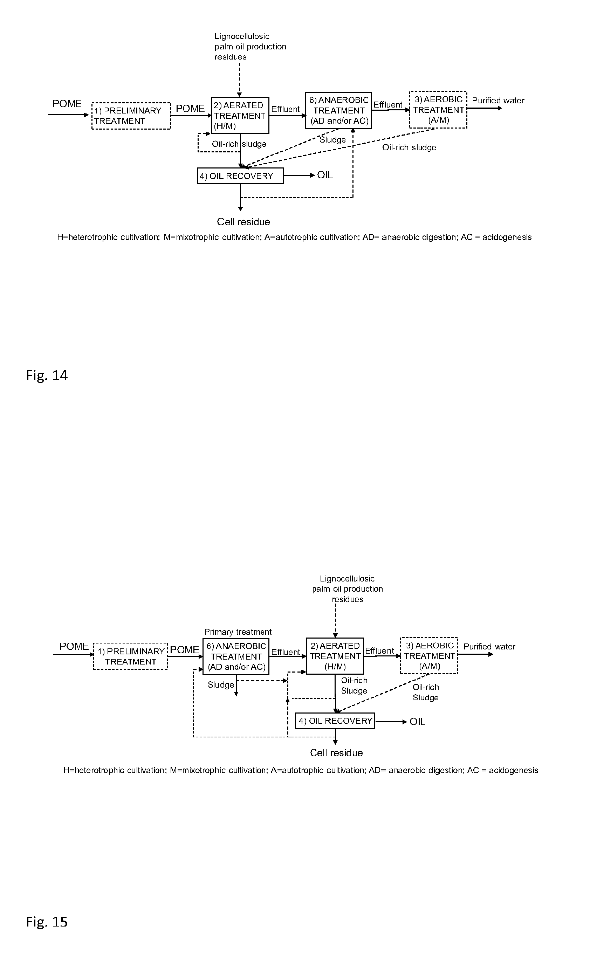

Scheme III presents the treatment of POME using first aerobic, preferably aerated treatment including oil accumulating hetero/mixotrophic microorganisms followed by anaerobic treatment (6) and optionally aerobic treatment (3) using auto/mixotrophic microorganisms. Aerated treatment with microbial oil accumulation may be supplemented with lignocellulosic palm oil production residues or hydrolysates thereof. Oil recovery (4) is performed to oil-rich sludge from aerated treatment and optionally from aerobic treatment. A preferred embodiment is presented in Figure 14.

Scheme IV presents the treatment of POME using first anaerobic treatment (6) followed by aerobic, preferably aerated treatment including hetero/mixotrophic oil accumulating microorganisms (2) and optionally aerobic treatment using auto/mixotrophic microorganisms (3). Aerated treatment with microbial oil accumulation may be supplemented with lignocellulosic palm oil production residues or hydrolysates thereof. Oil recovery (4) is performed to oil-rich sludge from aerated treatment and optionally from aerobic treatment. A preferred embodiment is presented in Figure 15.

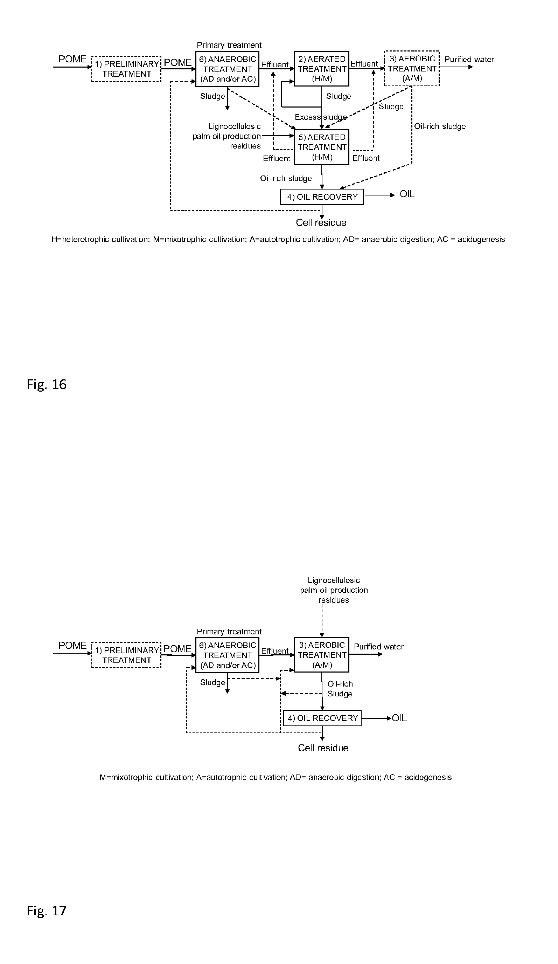

Scheme V presents the treatment of POME using first anaerobic treatment (6) followed by aerobic, preferably aerated treatment including hetero/mixotrophic microorganisms (2) and optionally aerobic treatment using auto/mixotrophic microorganisms. (3) Excess sludge from aerated treatment is fed to second aerobic, preferably aerated process, which uses hetero/mixotrophic oil accumulating microorganisms. Second aerated treatment process (5) is fed with lignocellulosic palm oil production residues or hydrolysates thereof to increase lipid production. Oil recovery (4) is performed to oil-rich sludge from aerated treatment and optionally from aerobic treatment. A preferred embodiment is presented in Figure 16.

Scheme VI presents the treatment of POME using first anaerobic treatment (6) followed by aerobic treatment including auto/mixotrophic oil accumulating microorganisms (3). Aerobic treatment with microbial oil accumulation may be supplemented with lignocellulosic palm oil production residues or hydrolysates thereof. Oil recovery (4) is performed to oil-rich sludge from aerobic treatment. A preferred embodiment is presented in Figure 17.

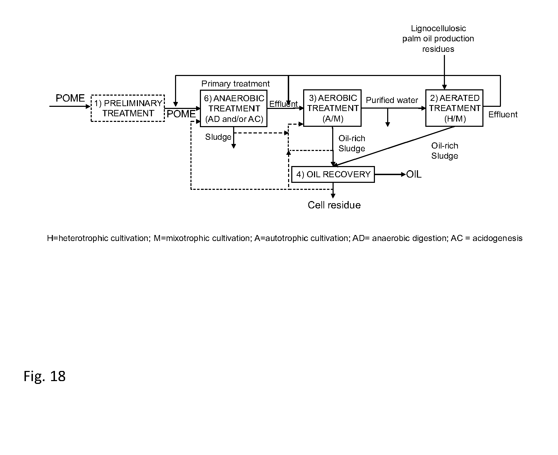

Scheme VII presents the treatment of POME using first anaerobic treatment (6) followed by aerobic treatment including auto/mixotrophic oil accumulating microorganisms (3) and aerobic, preferably aerated treatment including hetero/mixotrophic oil accumulating micro-organisms (2). Effluent from aerated treatment is recirculated to anaerobic and/or aerobic treatment. Aerated treatment with microbial oil accumulation may be supplemented with lignocellulosic palm oil production residues or hydrolysates thereof. Oil recovery (4) is performed to oil-rich sludge from aerobic treatment and aerated treatment. A preferred embodiment is presented in Figure 18.

In order to maximise lipid production, the supplementation of palm oil production residues with other materials may be feasible in order to adjust the carbon/ nitrogen (C/N)-ratio of cultivation medim. According to one embodiment of the invention, the lipid production by microorganisms, mono cultures or mixed cultures of microorganisms, cultivated in POME is increased by the addition of carbon compounds, such as carbohydrates, derived from lignocellulosic palm oil production residues. According to one embodiment of the invention, EFB or EFB hydrolysate is fed to in order to adjust the C/N ratio in cultivation medium to improve lipid production by microorganisms. Alternatively or in addition, other palm oil production residues, of fractions thereof, such as palm fruit fibre (mesocarp fibre), palm kernel shell, palm kernel cake, oil palm wood (e.g.. trunks, branches, roots) or fractions thereof, or oil palm fronds (leaves). or POME soilds can be used to adjust the C/N ratio in cultivation medium to improve lipid production by microorganisms.

According to one embodiment of the invention, microbial sludge from the treatment of palm oil mill effluent, such as excess sludge from aerobic pond, is taken to an aerated pond or reactor and fed with palm oil production residues in order to accumulate lipids.

According to one embodiment of the invention the primary treatment of POME is performed first as aerobic treatment in order to decrease the organic content of POME and the sludge from anaerobic treatment, such as excess sludge, is led to lipid accumulation process where lignocellulosic palm oil production are fed. Preferably, the influent stream containing palm oil production residues has high C/N -ratio in order to facilitate oil accumulation by microorganisms in the sludge. The supplementation of sludge with carbon sources increases the lipid content of microbial sludge or biomass.

The supplementation of organic carbon sources, such as lignocellulosic palm oil residues, to the treatment of POME increase the lipid production and lipid content of microbial biomass or sludge, while it still enables efficient decrease of COD in effluent. The organic load as COD, in particular as soluble COD in POME treatment with microorganisms under aerobic and/or aerated treatment(s) with supplementation of organic carbon sources can be decreased at least 5%, more prefably at least 15%, more preferably at least 20%, more preferably at least 30%, more preferably at least 40%, more preferably at least 50%, more preferably at least 60%, more preferably at least 70%, more preferably at least 80%, even more preferably at least 90% compared to the initial COD content in POME, i.e. the COD content in fresh waste water from the palm oil production process.

According to one embodiment of the invention autotrophic and heterotrophic oil production is combined in the treatment of palm oil mill residues.

Effluent from heterotrophic oil production process from palm oil mill residues is led to auto-and/or mixotrophic oil production process. Oil extracted cell residue or fraction thereof from heterotrophic and/or auto- and/or mixotrophic oil production process can be recycled to heterotrophic oil production process.The raw materials for the microbial oil production according to the invention comprise palm oil production residues. The palm oil production residues can be supplemented with any other organic raw materials. Preferably, at least 50 wt-%, preferably at least 60 wt-%, more preferably at least 70 wt-% of the organic raw materials used for oil production are palm oil production residues. Other raw materials used as supplements with palm oil residues include but are not limited to any lignocellulosic materials including woody plants or non-woody, herbaceous plants or other materials containing cellulose and/or hemicelluloses, such as stover, sugarcane bagasse; dedicated energy crops, such as switchgrass, *Miscanthus,* reed canary grass, willow, water hyacinth; wood materials or residues including e.g. sawmill and pulp and/or paper mill residues or fractions, such as hemicellulose, spent sulphite liqueur, waste fibre and/or primary sludge; moss or peat, microorganisms or municipal paper waste. Also low lignin materials, materials, such as macroalgae or microalgae biomass can be used. In addition, the materials can be also hemicellulose or cellulose fractions from industrial practises. Materials can be agricultural residues, such as wheat straw, rice straw, sugar cane bagasse, corn stover, sugar beet pulp, chaff, hulls. In addition, palm oil mill residues can be supplemented with biodiesel production residues, such as glycerol. In addition, palm oil mill residues can be supplemented with any starch or sugar materials, such as, but not limited to, starch, such as corn starch, wheat starch, potato starch, cassava starch etc.; or sugars, such as molasses from sugar cane or sugar beet.

Pre-treatment, e.g. hydrolysis of the lignocellulosic palm oil production residues, such as EFB, POME solids, palm fruit fibre, kernel shell or oil palm wood, can be performed by any known method in order to improve the digestibility of polymeric sugar hydrolysing enzymes. The pre-treatment can include the fractionation (separation) of hemicellulose and cellulose and possible lignin by any known method. Choosing the correct pretreatment method is to a large extent dependent on the type of lignocellulosic feedstock to be used in the process. There are several methods/technologies that are only suitable for certain type of raw material. Preferably, the separation of hemicellulose and/or cellulose is done with a method that produces hydrolysates which do not inhibit the growth of lipid producing microorganisms. The hemicellulose and cellulose fractions fed to the lipid production process may contain sugars in polymeric form.

The methods for the hydrolysis of lignocellulose in palm oil production residues comprise for example hot water extraction, acid treatment, steam explosion, ammonia assisted pre-treatment, enzymatic hydrolysis or Organosolv-treatment.

The enzymatic hydrolysis may not be required to lignocellulose fraction containing cellulose, hemicellulose and lignin prior to feeding lignocelluse or fractions thereof to a process that utilizes microorganisms capable of producing exoenzymes that hydrolyse polymeric sugars in lignocellulosic materials. These microorganisms are also capable for accumulation of lipids. In one embodiment of the invention, the palm oil mill residues are not pre-treated, e.g. hydrolysed, or de-polymerized before feeding to microbial oil production process utilizing single microorganism strain or mixed cultures. The residues, however, are possibly hygienized, such as pasteurized, before feeding to the oil production process.

In one embodiment of the invention, oil accumulating microorganisms, such as yeasts, filamentous fungi (moulds), heterotrophic algae or bacteria, are cultivated heterotrophically with palm oil production residues. In another embodiment of the invention, microorganisms, such as algae, are cultivated mixotophically on palm oil production residues.

In one embodiment of the invention, autotrophic microorganisms, such as algae and cyanobacteria, are cultivated for oil production on palm oil mill effluent and optionally other palm oil production residues.

Mixed cultures of two or more genera, species or strains of microorganisms are also within the scope of this invention.

In one embodiment of the invention microbial communities (mixed cultures) are used in the treatment of POME and optionally other palm oil production residues: The communities can accumulate oil from POME and optionally other palm oil production residues during the treatment.

The treatment of POME and possibly other palm oil production residues and oil accumulation by heterotrophic microorganisms occurs typically in aerated reactors. The reactors can be of any kind e.g. closed tanks (not open to air), such as fermentors, or ponds open to air alike aeration ponds used in activated sludge processes.

Mixed cultures can comprise bacteria, yeasts, filamentous fungi and/or algae and other microorganisms. Mixed communities can be grown heterotrophically, autotrophically or mixotrophically. It was surprisingly found that oil accumulating microorganisms can be found and enriched from POME. Lipid accumulating mixed cultures can be enriched from POME or from waste water treatment systems treating POME. Lipid accumulating mixed cultures can be also enriched from the environment, from industrial processes or from waste or waste water treatment systems. Lipid accumulating mixed cultures can also be made artificially by introducing (inoculating) known lipid accumulating microorganisms, such as bacteria, yeast, filamentous fungi, and/or algae to existing mixed communities, such as those in POME treatment. The inoculation of known microorganisms with desired properties to a mixed microbial cultures is sometimes called as bioaugmentation. Preferably, yeasts, filamentous fungi and/or Streptomyces bacteria are used in bioaugmentation. Further, lipid accumulating mixed cultures can be made artificially by co-cultuturing two or more microorganisms comprising at least one microorganism capable of lipid accumulation.

According to the invention any (one or more) microorganisms that is/are capable of producing lipids can be used. These organisms include algae, bacteria, fungi, such as yeasts and filamentous fungi, algae and archaea. Lipid producing organisms may naturally produce lipids or produce lipids after genetic modification. Lipid producing organisms include, but are not limited to the following organisms:

Filamentous fungal species belonging to the following genera comprising: *Absidia, Aspergillus, Blakeslea, Chaetomium, Cladosporium, Claviceps, Cladosporidium, Cunninghamella, Emericella, Entomophthora, Fusarium, Gibberella, Glomus, Humicola,* Malbranchea, *Mucor, Mortierella, Paecilomyces, Penicillium, Puccia, Pythium, Rhizopus, Saprolegnia, Trichoderma, Thermomycs, Ustilago* ja *Zygorhynchus* -genera, such as *Absidia spinosa,* species belonging to the genus *Aspergillus,* such as *A. ficheri, A. flavus, A. nidulans, A. ochraceus, A. oryzae* and *A. terrius, Blakeslea trispora, Chaetomium globosum, Cladosporidium herbarum, Claviceps purpurea,* species belonging to the genus *Cunninghamella,* such as *C*. *echinulata, C*. *japonica* and *C*. *elegans, Entomophthora coronata, Fusarium bulbigenum, Fusarium graminearum, Fusarium sp., Gibberella fujikuroi, Glomus caledonius, Humicola lanuginosa,* species belonging to the genus *Mucor,* such as *M. circinelloides, M. plumbeus* and *M*. *rouxii,* species belonging to the genus *Mortierella* such as *M*. *isabellina, M. alpina* and *M*. *ramanniana,* species belonging to the genus *Penicillium* such as *P. javanicum, P. lilacinum, P. spinulosum* and *P. soppii, Puccia coronata, Pythium ultimum, Rhizopus delemar, Rhizopus oryzae*, *Ustilago zeae*, *Zygorhynchus moelleri* and *Malbranchea pulchella, Myrothecium sp., Sclerotium bataticola, Pellicularia practicola, Sphacelothea reiliana, Tyloposporidium ehren bergii, Achyla americana, Lepista nuda, Tilletia controversa* and *Cronartium fusiform.*

Microalgal species belonging to the genera comprising *Dunaliella, Chlorella, Achnantes, Amphiprora, Amphora, Ankistrodesmus, Boekolovia, Boekolovia, Botryococcus*, *Brachiomonas, Carteria, Chaetoceros*, *Chlorococcum*, *Crypthecodinium, Cryptomonas, Chrysophaera, Cricophaera, Cyclotella, Eremosphaera, Ellipsoidon, Euglena, Franceia, Fragilaria, Gleocapsa, Gleothamnion, Haematococcus, Hymenomonas, Chlamydomas, Isochrysis, Lepocinclis, Micractinum, Ocromonas, Oocystis*, *Oscillatoria, Pascheria, Phagus, Phormidium, Platymonas, Pleurochrysis, Pavlova, Prototheca, Phaeodactylum, Pseudochlorella, Parachlorella, Pyramimonas, Bracteococcus, Scenedesmus, Skeletonema, Chaetoceros*, *Melosira. Monoraphidium, Nitzschia, Nannochloropsis, Navicula, Neochloris, Nannochloris, Nephroselmis, Sarcinoid, Spirogyra, Stichococcus*, *Scihizochytrium, Scrippsiella, Tetraedron, Thalassiosira, Thraustochytrium, Volvox, Viridiella, Ulkenia* and *Tetraselmis.*

Bacteria belonging to the genera comprising *Acinetobacter, Actinobacter, Agmenellum, Alcanivorax, Aerogenes, Anabaena, Anabaenopsis, Arthrobacter, Arthrospira, Bacillus, Clostridium, Coccochloris*, *Cupriviadus, Dietzia, Dermocarpa, Gordonia, Escherichia, Flexibacterium, Micrococcus*, *Microcystis, Mycobacterium, Nocardia, Nostoc, Nodularia, Oscillatoria, Phormidium, Plankthothrix, Plectonema. Pseudomonas, Pyrobotrus, Ralstonia, Rhodococcus*, *Rhodomicrobium, Rhodopseudomonas*, *Spirulina, Synechococcus*, *Synechocystis, Shewanella, Shigella, Streptomyces, Vllautersia, Vibrio* and *Xenococcus*, especially *Rhodococcus opacus*, *Acinetobacter, Nocardia* or *Streptomyces,* such as *Streptomyces roseosporus*, *Streptomyces griseus, Streptomyces albus, Streptomyces peucetius, Streptomyces aureofaciens Streptomyces lividans, Streptomyces coelicolor*, *Streptomyces hygroscopicus, Streptomyces avermitilis, Streptomyces milbemycinius,* and *Streptomyces lydicus.*

Yeasts belonging to the following genera comprising *Clavispora, Deparyomyces, Pachysolen, Kluy-veromyces, Galactomyces, Hansenula, Saccharomyces*, *VValtomyces, Endomycopsis, Cryptococcus*, such as *Cryptococcus curvatus, Rhodosporidium,* such as *Rhodosporid-ium toruloides, Rhodotorula,* such as *Rhodotorula glutinis, Yarrowia,* such as *Yarrowia lipolytica, Pichia,* such as *Pichia stipitis, Candida,* such as *Candida curvata, Lipomyces*, such as *Lipomyces starkeyi* or *Lipomyces lipofer* and *Trichosporon,* such as *Trichosporon cutaneum* or *Trichosporon pullulans.*

In one embodiment of the invention, mixed microbial cultures are used that contain at least one microorganism capable of hydrolysing polymeric sugars, such as cellulose and/or hemicellulose, to sugar monomers and at least one microorganism capable of accumulating lipids using sugars as carbon source.

In preferred embodiments of the invention oleaginous, lipid producing organisms that are capable of utilizing polymeric hemicellulose or cellulose due to exoenzymes, such as hemicellulases, for example xylanases, galactosidases or mannanases, or cellulases are used. These organisms include but are not limited to bacteria, such as *Streptomyces* or *Bacillus,* filamentous fungi, such as *Aspergillus, Fusarium, Humicola, Penicillium, Phanerochaete*, *Rhizopus,* or *Trichoderma,* such as *A*. *niger, A. terreus, A. oryzae*, *A*. *nidulans, F. oxysporum, Phanerochaete chrysosporium, Rhizopus oryzae* or *Trichoderma reesei*, yeasts, such as *Cryptococcus* or *Trichosporon,* such as *Cryptococcus albidus, Trichosproron cutaneum.* Oleaginous microorganisms that are genetically modified to be able to utilize or to increase utilization of polymeric sugars in hemicellulose or cellulose are also within the scope of the invention.

### Lipid production process

Microbial lipid production or accumulation from palm oil production residues can be performed with any known method or a method developed in the future. The cultivation of oil accumulating microorganisms can be performed in any kind of bioreactors.

According to one embodiment of the invention, the heterotrophic oil production from palm oil production residues in carried out in an aerated bioreactor, a fermentor, in which microorganisms, such as yeasts, filamentous fungi, bacteria or algae, or mixed cultures thereof are cultivated. The microorganism culture for heterotrophic oil production from palm oil production residues can comprise one of more species of microorganisms, preferably bacteria, such as *Streptomyces;* yeasts or filamentous fungi. According to one embodiment of the invention, the treatment of POME and possibly other palm oil production residues and oil accumulation by single microbial strain is performed in an aerated closed tank, such as fermentor.

According to one embodiment of the invention, the palm oil production residues are hygienized, such as pasteurized before feeding to the heterotrophic oil production process. According to one embodiment of the invention, the palm oil production residues, or fraction of the residues is sterilized before feeding to heterotrophic oil production process. Preferably, the palm oil production residues are not sterilized before feeding to heterotrophic oil production process.

By "sterilization" it is typically meant that the residue material or medium comprising the residue material is treated with high temperature, usually at 121 °C, or higher, for at least 15 minutes, or for at least 20 minutes, typically at 121 °C for at least 20 minutes.

"Pasteurization" refers to the heating of a waste or residue material or medium comprising waste or residue material at 60 °C - 75 °C for 2 - 30 minutes.

According to one embodiment of the invention, the heterotrophic microbial lipid production process includes cultivation of microorganisms in aerated bioreactors, such as in fermentors in submerged cultivation. Microorganisms are grown in a liquid culture medium comprising carbon and energy sources, such as palm oil production residues, and possible added macro- and micronutrients. The cultivation is typically performed under aerobic, preferably aerated conditions, where the concentration of dissolved oxygen is at least 0.001 mg/l, i.e. above 0 mg/l. Cultivation can be performed e.g. as batch cultivation, fed-batch cultivation, continuous cultivation. Cultivation can be also performed in a cascade process. In cultivation, microorganisms are let to grow and accumulate lipids intracellularly. Some microorganisms can also be able to excrete the lipids to culture medium.

The microbial lipid production process from palm oil production residues can be carried out also in reactors, where the amount of free water is low or where the production is carried out on a solid or semisolid surface.

In various embodiments of the invention, oil, or precursors for oil, may be recovered from cell biomass or culture broth using any method known in the art or developed in the future. For example, microorganisms are separated from the medium using a filtration or decanting techniques. Alternatively, centrifugation with industrial scale commercial centrifuges of large volume capacity are used to separate the desired products. The microbial biomass separated from the medium is then led to oil recovery.

### Culture processes for treatment of POME and optionally other palm oil production residue(s) and oil accumulation

According to one embodiment of the invention, the oil accumulation and/or recovery from palm oil production residues is performed in POME treatment by mixed microbial cultures. The treatment of POME, i.e. reduction of organic load in POME and optionally other palm oil production residue(s), by mixed cultures can thus be combined with the oil production and/or recovery of oil by microorganisms. The microbial biomass containing oil can be collected from waste water treatment system by any method, such as by using methods typically used in waste water treatment processes, e.g. flocculation, flotation or sedimentation. The microbial biomass or sludge separated from the waste water is then led to oil recovery.

According to one embodiment of the invention, the oil production by mixed microbial cultures is performed in open, preferably aerated ponds, such as in aeration ponds typically used in activated sludge waste water treatment plants. Mixed microbial cultures can be inoculated by micro-organisms capable of oil accumulation (bioaugmentation), such as oleaginous microorganisms of yeasts, filamentous fungi, bacteria and/or algae.

The invention is not limited to the type, amount or size of aerobic ponds used for lipid accumulation by mixed cultures. Oil accumulation by mixed cultures can be performed in any kind of aerated tanks, reactors or ponds used in waste water treatment today or new types of reactors invented in the future. The oil accumulation reactors or ponds can comprise one or several ponds arranged in any configuration such as in series or parallel to each other. Typically, the aerobic reactors or ponds for oil accumulation are aerated by sparging air or oxygen gas. According to the present invention aerobic ponds also include ponds that are not mechanically aerated by purging air or oxygen. According to one embodiment of the invention, the treatment of POME and optionally other palm oil production residue(s) oil accumulation is performed by mixotrophic microorganisms in shallow ponds or photobioreactors without aeration. Therefore, the invention covers the utilization of any kinds of aerated or aerobic ponds or reactors for the accumulation of oil from palm oil residues.

According to this disclosure, the aeration in the ponds is typically organized such a way that the dissolved oxygen concentration in the liquid phase is at least 0.001 mg/l i.e. above 0 g/l. More preferably, the aeration is arranged that way that the dissolved oxygen concentration is above 0.5 mg/l, more preferably above 2 mg/l, even more preferably above 5 mg/l.

In order to maximise the lipid content of microbial biomass or sludge, the harvesting of sludge or biomass need to be carried out at right time, otherwise the lipids can start to degrade.

In mixed culture process treating POME, lignocellulosic residues from palm oil production or hydrolysates thereof can be used to adjust C/N ratio of the POME to improve oil accumulation by heterotrophic and/or mixotrophic microorganisms. Other lignocellulosics materials or hydrolysates thereof than those from palm oil production, as well as starch or molasses can be used to adjust C/N ratio as well.

According to one embodiment of the invention, the microbial biomass for the lipid accumulation is grown with palm oil mill effluent. The lipid production and accumulation in micro-organisms is facilitated by increasing the C/N-ratio of the feed by lignocellulosic palm oil production residues such as EFB, palm fibre or POME concentrate or POME solids or hydrolysates thereof.

In one embodiment of the invention, the POME is concentrated before use in the cultivations until the dry weight of POME solids is at least 10%, preferably 20% to 100%, more preferably 30% to 60 %.

According to one embodiment of the invention, the autotrophic and/or mixoptrophic micro-organisms are grown on POME and optionally other palm oil production residue(s) in shallow open ponds, typically less than 1 m deep, that are typically used in cultivation of algae.

According to another embodiment of the invention, the autotrophic and/or mixotrophic microorganisms are cultivated on POME and optionally other palm oil production residue(s) in closed photobioreactors for oil accumulation. Gases containing concentrated CO₂, such as smoke gases, or CO₂ from biogas or heterotrophic cultivation are preferably purged to shallow ponds or photobioreactors to provide carbon dioxide to autotrophic microorganisms. Autotrophic or mixotrophic oil accumulation process is preferably fed with smoke gases from power plant, e.g. from power plant located in palm oil mill.

According to one embodiment of the invention, first heterotrophic oil production is performed on palm oil production residues. The microorganism cells or sludge is collected from the heterotrophic oil production. After that treatment auto or mixotrophic oil production with algae and/or cyanobacteria is performed on the effluent from heterotrophic oil accumulation process.

According to another embodiment of the invention, first auto and/or mixotrophic oil production process is performed on palm oil mill effluent. After that treatment heterotrophic oil production is performed on the effluent and/or solid residue from auto and/or mixotrophic oil accumulation process. Heterotrophic oil production process treating residues from auto and/or mixotrophic oil accumulation process can be supplemented with lignocellulosic palm oil production residues.

POME is typically acidic and its pH varies typically within the range from 3.0 to 6.5.POME. During the aerobic treatment with microorganisms, the pH of POME typically increases above 7, even up to 9 or above.

According to one embodiment of the invention, in order to increase lipid production by microorganisms during aerobic waste water treatment of POME, lipid accumulating fungi, such as yeasts and/or filamentous fungi are enriched into the sludge in the waste water treatment system.

According to one embodiment of the invention, the pH in the treatment of POME is adjusted from 3.5 to 7 in order to promote the growth of lipid accumulating fungi in order to enrich them. Prefably, pH is adjusted between 3.5 and 6 to enrich lipid accumulating fungi. More preferably, the pH is adjusted between 4 and 5.5 to enrich lipid accumulating fungi. The adjustment of pH can be done by any method.

According to one embodiment of the invention, the lipid accumulating microorganisms are enriched in the treatment of POME by addition of organic carbon sources to the treatment system. According to one embodiment of the invention, the lipid accumulating microorganisms are enriched by addition of lignocellulosic palm oil production residues, or hydrolysates thereof, to the treatment system.

According to one embodiment of the invention, the lipid accumulating fungi are enriched in the aerobic POME treatment system by adjusting the pH, preferably between 3.5 to 6, and adding organic carbon sources, such as lignocellulosic palm oil mill residues or hydrolysates thereof to the POME treatment. The addition of carbon, such as lignocellulosic carbon sources to the POME treatment also increases the lipid production by microorganisms.

According to yet another embodiment of the invention, the pH of in the aerobic waste water treatment system of POME is decreased by adding acidic waste or residue streams, such as lignocellulosic palm oil mill residue hydrolysates to the treatment system. Typically acidic hydrolysates of lignocellulosic materials can be obtained in treatments, such as acid treatment, steam explosion or hot water extraction of lignocellulosic material. The decrease of pH in POME treatment system can increase the growth of lipid accumulating fungi and the increase of carbon improve the lipid production by microorganisms.

According to one embodiment of the invention, the lipid production by microorganisms from palm oil production residues occurs in temperature range of mesophilic microorganisms, between 10 to 45 °C, preferably between 25 to 45 °C. According to another embodiment of the invention, the lipid production by microorganisms from palm oil production residues occurs in temperature range of thermophilic microorganisms, above 45 °C, preferably between 45 and 80 °C.

According to one embodiment of the invention, hemicellulose hydrolysates are produced from lignocellulosic palm oil mill residues. The separation of hemicellulose from lignocellulose can be made by any known method or method developed in the future, such as hot water extraction. The hemicellulose hydrolysate is fed to microbial lipid production process, such as to POME treatment process with lipid accumulation by microorganisms. Optionally, enzymatic hydrolysis is performed to hemicellulose before feeding to lipid accumulation process. The cellulose and lignin fraction after separation of hemicellulose can be used for various purposes, e.g. the cellulose can be separated from lignin and used for pulp and paper making applications. The cellulose fraction can also be fed to microbial lipid production process, such as to POME treatment process with lipid accumulation by microorganisms. The cellulose can be separated for lignin and/or enzymatically digested before feeding to microbial lipid production process. The lignin fraction can be combusted in boiler to provide electricity and heat or refined to chemicals and/or biofuels.

According to one embodiment of the invention, at least part of the solids and/or oil from POME is recovered, e.g. by filtration, and after this the POME solids other than oil is fed to a pre-treatment process where the sugar polymers are at least partly hydrolysed. The POME solids can be hydrolysed together with other lignocellulosic residues from palm oil production or other lignocellulosic materials. The hydrolysate is fed to a process containing POME filtrate (essentially free of solids) as culture liquid and lipid accumulating micro-organisms. Microorganisms are let to accumulate lipids and after that oil-rich microbial biomass is separated from the cultivation liquid. After this, oil is recovered from the microorganism biomass.

According to one embodiment of the invention, lignocellulosic residues from palm oil production are used as raw materials in the production of oil by microorganisms without the use of palm oil mill effluent or with the use of palm oil mill effluent as a combination with some other suitable liquid, as cultivation liquid.

### Oil recovery

The oil recovery from microbial biomass or sludge collected from oil production (oil accumulation) process using palm oil production residues can be performed with any suitable method. The microbial biomass or microorganism sludge from oil production process may be dried prior to oil recovery.

In various embodiments of the invention microorganism cells may be disrupted to facilitate the separation of oil and other components. Any method known for cell disruption may be used, such as ultrasonication, osmotic shock, mechanical shear force, cold press, thermal shock, enzyme-catalyzed or self-directed autolysis. Oil can be recovered from cells by extraction with organic solvents or by any method known in the art or developed in the future. Typically, non-polar solvents, such as hexane or heptane can be used in the oil recovery from microorganisms cells. Any other non-polar solvent(s), or combinations of solvent with different polarities such as polar and non-polar solvents can be used.

### Use of lipids produced

Purpose for the lipids or a fraction of the lipids produced with the method described is not restricted to any specific application. The lipids produced may be used for the same purposes that palm oil is used. The lipids can be used, e.g. but are not limited to, in food or feed purposes, in cooking, as nutraceuticals, in production of soap or detergents, in production of cosmetics as chemicals or raw material for production of chemicals, as biofuel or as raw material for the production of biofuel or as lubricants as base oils for lubricants (lubrication oils) or as a starting material for production of base oils for lubricants.

Preferably, the lipids recovered from microbial biomass consisting of single microbial strains or mixed microbial cultures with the method described in this invention are used as feedstock for the production of biodiesel, renewable diesel, jet fuel , gasoline or lubricants. Biodiesel comprises of fatty acid alkyl esters, typically fatty acid methyl esters, and is typically produced by transesterification. In transesterification, the acylglycerols are converted to long-chain fatty acid alkyl (methyl, ethyl or propyl) esters. Renewable diesel refers to fuel which is produced by hydrogen treatment (hydrogen deoxygenation, hydrogenation or hydroprocessing) of lipids. In hydrogen treatment, acylglycerols are converted to corresponding alkanes (paraffins). The alkanes (paraffins) can be further modified by isomerization or by other process alternatives. Renewable diesel process can also be used to produce jet fuel and/or gasoline. In addition, cracking of lipids can be performed to produce biofuels. Further, lipids can be used as biofuels directly in certain applications.

"Biofuel" refers to solid, liquid or gaseous fuel mainly derived from biomass or biowaste and is separated from fossil fuels, which are derived from the organic remains of prehistoric microorganisms, plants and animals.

According to EU directive 2003/30/EU "biodiesel" refers to a methyl-ester produced from vegetable oil or animal oil, of diesel quality to be used as biofuel. More broadly, biodiesel refers to long-chain alkyl esters, such as methyl, ethyl or propyl-esters, from vegetable oil or animal oil of diesel quality. Biodiesel can also be produced from microorganism lipids, whereby microorganism lipid can originate from a bacterium, a fungus (a yeast or a mold), an algae or another microorganism.

"Renewable diesel" refers to a fuel which is produced by a hydrogen treatment of lipids of an animal, vegetable, plant or microorganism origin, or their mixtures, whereby microorganism lipid can originate from a bacterium, a fungus (a yeast or a mold, i.e. filamentous fungus), an algae or another microorganism. Renewable diesel can be produced also from waxes derived from biomass by gasification and Fischer-Tropsch synthesis. Optionally, in addition to hydrogen treatment, isomerization or other processing steps can be performed. Renewable diesel process can also be used to produce jet fuel and/or gasoline. The production of renewable diesel has been described in patent publications EP 1396531, EP1398364, EP 1741767 and EP1741768.

Biodiesel or renewable diesel may be blended with mineral oil based diesel. Suitable additives, such as preservatives and antioxidants may be added to the fuel product.

"Lubricant" refers to a substance, such as grease, lipid or oil, that reduces friction when applied as a surface coating to moving parts. Two other main functions of a lubricant are heat removal and to dissolve impurities. Applications of lubricants include, but are not limited to uses in internal combustion engines as engine oils, additives in fuels, in oil-driven devices such as pumps and hydraulic equipment, or in different types of bearings. Typically lubricants contain 75-100% base oil and the rest is additives. Suitable additives are for example detergents, storage stabilizers, antioxidants, corrosion inhibitors, dehazers, demulsifiers, antifoaming agents, cosolvents, and lubricity additives (see for example US 7,691,792). Base oil for lubricant can originate from mineral oil, vegetable oil, animal oil or from a bacterium, fungi (a yeast or a mold), an algae or another microorganism. Base oil can also originate from waxes derived from biomass by gasification and Fischer-Tropsch synthesis. Viscosity index is used to characterise base oil. Typically high viscosity index is preferred.

### Centralized or de-centralized facilities

The microbial oil production from palm oil mill residues can be performed next to a palm oil mill. The microbial oil production can also utilize palm oil mill residues from one or several palm oil mills. According to one embodiment of invention, the microbial oil production is performed in a centralized facility that utilizes residues from several mills.

According to one embodiment of the invention, the oil production process uses POME from one mill as cultivation liquid and in addition lignocellulosic residues or their hydrolysates and/or possibly microorganism sludges from at least two palm oil mills. In another embodiment the process is used to treat POME from one mill while the addition of lignocellulosic palm oil production residues from several mills increases the oil production capacity of the microbial oil production process.

According to one embodiment of the invention, POME or fractions thereof is collected from several mills for microbial oil production in a centralized facility where the POME treatment and oil accumulation by microorganism is performed. E.g. POME is dehydrated to 20-100 % dry matter content before transportation. According to one embodiment of the invention, at least part of the solids and/or oil from POME are recovered, e.g. by filtration and dehydrated to 20-60 % dry matter content before transportation to centralized facility of microbial oil production.

### Treatment of residual biomass and effluent

The biomass residue from oil production, i.e. biomass (cell) residue after oil recovery, can be used for various purposes. According to one embodiment of the invention, the oil-extracted biomass residue is used as animal feed. According to another embodiment of the invention, the biomass residue is used as fertilizer and/or mulch in the oil palm plantations. Before the use as a fertilizer or mulch, the cell residues can be possibly composted.

According to yet another embodiment of the invention, the oil-extracted biomass residue is treated with anaerobic digestion to produce biogas. The biogas is preferably collected and potentially used for production of electricity and/or heat or flared. According to one embodiment of the invention, methane is purified from the biogas produced and liquefied and sold as a product. The digestate, e.g. solid digestate and effluent, from anaerobic digestion can be used as a fertilizer and/or mulch in oil palm plantations. The digestate can be composted before use as a fertilizer and/or mulch.

According to one embodiment of the invention, at least part of the anaerobic digestion effluent (i.e. digestate) is used as nutrient source in hetero-, auto- or mixotrophic oil production process.

According to one embodiment of the invention, the oil-extracted biomass residue is combusted. The ash from combustion may be used as a fertilizer in oil palm plantations.

According to one embodiment of the invention, the oil-extracted cell residue is recirculated at least partly back to oil production process.

The effluent from microbial oil production process can be used potentially as fertilizer and/or for irrigation in oil palm plantations. Alternatively, the effluent is treated in waste water treatment unit, which for example comprises aerobic and/or anaerobic treatment units. The biogas potentially generated in waste water treatment is collected and used for production of electricity and heat or flared.

According to yet another embodiment of the invention, the effluent from microbial oil production process is used at least partly for the composting of palm oil mill residues and/or oil-extracted microbial residues.

According to one embodiment of the invention, the effluent (supernatant) from microbial oil production is at least partly recirculated back to oil production process.

### Lipid production

The present invention was exemplfied by using Streptomyces strains as lipid producing microorganisms in cultivations. The *Streptomyces* lipids are beneficial for the production of biofuel. *Streptomyces* fatty acids typically contain methyl-branched chain fatty acids, which are beneficial for biofuel and lubricant applications. Branched fatty acids have a broader liquidity range, making them of interest for low-temperature applications and their low surface tension causes good spreadability (Gunstone et al. 2007). Fatty acid branching improves the cold properties of biofuels, such as biodiesel or renewable diesel. The *Streptomyces* oil has relatively high saturation degree (contains high amounts of saturated fatty acids). This property is an advantage especially for the renewable diesel process, since fatty acid saturation decreases the amount of hydrogen needed in hydrogenation. High saturation degree of lipids may also improve the stability and storability of oil, and reduce the need of antioxidants in oil storage. Further, the main fatty chain lenghts are mainly from C14 (14 carbons) to C18 (18 carbons), which is advantageous for the utilization in diesel applications.

Lipids produced can be used as base oils for lubricants (lubrication oils) or as a starting material for production of base oils for lubricants. Steptomyces oil contain squalene or squalene derivates (Gräfe et al. 1985; Olukoshi and Packter 1994). Especially, squalene and squalene derivates are suitable for uses in lubricant applications. However, other lipids by *Streptomyces* can be used for lubricant applications as well.

Biofuel which comprises the lipid composition produced according to the present disclosure may comprise advantageous properties for biofuel use. Such properties include for example fatty acid branching which improves cold properties. Fatty acid saturation is advantageous especially for renewable diesel production.

### Cultivations in the broth by Streptomyces

Streptomycetes can be cultivated for lipid accumulation in batch, fed batch mode as well as continuous mode, typically cultivation is performed in fed-batch mode. The form of first inoculation to start a cultivation cycle may be a culture on a solid media allowing the growth of streptomycetes, a mycelia stock, a lyophilized and preferably a spore suspension. The spore suspension is inoculated to submerged aerobic fermentation. The proceeding inoculum cultures may be made as submerged cultures in fermentors. To make a large volume cultivation, e.g. in 150 m³ to 1000 m₃ fermentation, several seeding stages may be carried out to gradually increase the cell mass.

Under controlled conditions in a medium containing pure or crude ingredients and sugars, such as glucose and/or starch as main carbon source, such as E05 medium, the *Strep*tomyces strains accumulated about 3 to 5 g/l of TAGs, as an average while the range of 1 g/l up to 8 g/l was obtained when 20g/L of dextrose and 20g/L of starch was used as carbon sources. The content of E05 medium is given in Example 2. Accumulation of lipids started in E05 when protein was used to the growth, typically in 12-64 hours from the start of cultivation to be continued up to 144 hours.

In the examples it was shown that the optimal concentrations of nitrogen sources added to the medium may be dependent on the amount of carbon sources, such as sugars, added. At the cultivation conditions where temperature is in the range of 22-36 °C and oxygen is supplied by agitation and aeration in flasks or in controlled fermentor, the cultivation time for maximal lipid yields is typically from three to five days.

It was exemplified here that the improvement of TAG production in the *Streptomyces* strains using POME with lignocellulosic palm oil production residue(s), such as EFB and/or cell waste was 60 - 300 %, typically 100 - 200 % compared to the cultivation in a standard medium, such as E05 medium with 20g/L of dextrose and 20g/L of starch.

In various embodiments of the invention the concentration (titre) of TAGs was generally at least 5 kg/1000 litre of culture broth, varying from 1 to 8 kg/1000 litre with pure or crude ingredients and sugars, such E05 medium. In embodiments when palm oil production residues, such as POME and EFB, and cell waste were used 5 to 30 kg/1000 litre of TAGs was obtained.

The lipid profile characteristic to strains cultivated on pure sugar e.g. in E05 comprised typically about 70 to 95 wt-% of TAGs and about 5 to 30 wt-% of oligomers, diacylglycerols and monocylglycerols and free fatty acids. The fatty acids comprised typically branched and straight chain fatty acids. The fraction of methyl-branched fatty acids was between 20 to 90 wt-%, and typically between 40 to 70 wt-%. The majority of fatty acids determined were saturated.

In various embodiments of the invention the lipid fraction produced by streptomycetes, comprised mainly TAGs. This means that at least 20 wt-%, in some embodiments at least 50 wt-%, in some other embodiments at least 60 wt-%, yet in some further embodiments at least 70 wt-%, yet in some further embodiments at least 80 wt-%, of the lipid fraction is TAGs.

### Oil production by Streptomyces from palm oil production residues

Unexpectedly, palm oil mill production residues, POME and EFB, provided an efficient cultivation medium to *Streptomyces* strains for lipid production. In the present examples POME was used as a cultivation liquid into which other medium components were added in the range of 50-100 % (v/v). In the cultivation medium EFB was used in the range of 10 - 50 g/l. In a batch type cultivation where POME alone or with EFB is the only broth components, the *Streptomyces* strains were able to grow and and increase TAGs by at least 1 g/l and the highest value of TAGs was found in three days.

According to this invention in the cultivations can be used also waste material, such as cell waste or cell residues after oil recovery, in combination with palm oil mill residues.

In cultivation of *Streptomyces* strains in the medium E05 containing pure components, such as dextrose and starch, with only one exception, Pharmamedia, the accumulation of TAGs was 3 g/l in 4-6 days as an average, in E05 with less N-sources the TAGs of 5-8 g/l were obtained in 4-5 days. Replacing the sugar components, dextrose (20 g/l) and starch (20 g/l), that are C-sources for bacterial growth by POME and EFB in E05 broth gave increased TAGs accumulation as 5 g/l and the highest value was obtained in 3 days. The concentration of these wastes in the broth was varied as well as the composition of the wastes. However, since the wastes provided sufficient C-source to the strains, several different combinations were tested and it was shown that the use of POME and/or EFB was advantageous to accumulation of TAGs.

Cell waste derived from streptomycetes mycelia debris from previous cultivation cycle was used in the content of 10 - 20 g in I litre of POME and EFB (20 g/l of POME). The steady stage was observed after 12-72 h cultivation and accumulation of TAGs started in 48 h from the inoculation. Increase of TAGs was 10 - 15 g/l in three days providing maximal TAG content in 5 to 6 days. It is advantageous to add reused cells and EFB to the fermentation medium of POME. It gives additional and advantageous elements for cell growth and for TAGs accumulation by *Streptomyces.* The range of cell waste was typically 5 - 50 g/l to keep the media in liquid form and the amount of EFB or hydrolysates thereof may typically vary between 10 to 400 g/l as dry matter.

Though there are variations in waste materials, the growth of the strains was in the range that is typical to microbiological processes giving variations in PMV values from 10-50 % and the time for growing was between 12 to 72 hours.

Typically, cultivation temperature for *Streptomyces* may vary between 20-40°C while the optimal temperature is typically 28-30°C. It is possible to use high aeration and agitation for fermentation and a wide range of 0.1-1.5 vvm, 100-600 rpm, respectively was found to be useful. The inoculation by spores or by seed cultures gave the results that were in the range of +/- 20 %.

The improvement of TAG production by the strains using reduced nitrogen content in the start of cultivation, at the vegetative growth stage, was at least 4 g/l (from 1 to 5 g/l), and even 7 g/l (from 1 to 8 g/l) as compared to the cultivation in a standard E05 medium. The amount of optimal nitrogen content can very on the amount of carbon sources added.

The improvement of TAG production in the strains using POME with EFB and cell waste was 60 - 300 % compared to the cultivation in a standard E05 medium.

"Cell waste or cell residue" refers to a broth , such as spent culture medium used in the cultivation of microorganisms. It comprises the remaining solid residues after the use of the cell mass for a primary target, e.g. for extraction of small molecules or lipids. Cell waste can be also sludge formed in the microbiological waste water treatment.

Furthermore, in various embodiments of the invention the amount of total lipids was at least 10 % by the weight of dry cell mass, typically it was 20 - 80 % by the weight of dry cell mass.

The composition of the lipid profile may change when *Streptomyces* is cultivated on a medium comprising waste(s) or residues(s) instead of pure sugar, because lipid extraction may comprise lipids included in the waste material in the beginning (for example membrane phospholipids are extracted). In addition, microorganisms are able to uptake lipids from the cultivation medium and incorporate them to intracellular lipid inclusions.

In various embodiments of the invention the cultivation can be carried out in 1 to 14 days, typically in 3 to 10 days, preferably in 2 to 8 days.

As wastes or residues may contain lipids it is rational to follow up the use of the external lipids to determine the lipids formed by the bacterial strains. As described herein the utilization of external lipids was followed up by changes in solid biomass and changes in lipid content. It was shown that the biomass decreased up to 36-48 hours from the beginning of the cultivation and started to increase after that. The cycle time is dependant on the degree of processing of waste and the amount of waste materials used in the cultivation. Nevertheless, no remarkable differences were found after five days of cultivation when cultivation was followed up to 8 days.

The lipid yields (g_{lipids} / g_{glucose}) determined to pure sugar varied in the range of 8-55 %. The yields were determined based on the pure sugar and did not consider the sugar included in wastes and residues. Theoretical maximum of lipid yield from glucose is 33 %. However in practice, the maximum lipid yield is 20-22% (Ratledge and Cohen 2008).

Thus, lipid yields higher than 22 % indicate that lipids were generated or produced from waste and residue materials.

The present invention can be carried out by using *Streptomyces* strains deposited in several Microbial Culture Collections from which they are available to the public. Examples of suitable strains are *S*. *albus* DSM 40313, *S*. *lividans,* NRRL B16148, *S*. *coelicolor* DSM 40233, *S*. *hygroscopicus,* NRLL 5491, *S*. *aureofaciens,* DSM 40731, *S*. *griseus* ATCC 13273, *S*. *roseosporus* NRRL 11379, *S*. *avermitilis* NRRL 8165, S. *lydicus* ATCC 25470 and *S*. *peucetius P55* DSM 19075. The production of lipids can be enhanced for example by making the strains deficient for example of producing bioactive metabolites or lipases. The lipid production can be enhanced also by introducing into them genes of the lipid synthesis pathway.

The present invention was exemplified by using *Streptomyces roseosporus* and *Streptomyces albus,* more specifically strains G009 and G012.

**G012** is a *Streptomyces roseosporus* G011 strain carrying pIJEsco0958 plasmid. It does not produce detectable quantities of bioactive metabolites. Colony morphology is identical to that described for G011. The accumulation of TAGs was 12 g/l.

sco0958 is responsible for the terminal reaction in triacylglyceride (TAG) biosynthesis being an esterification of diacylglycerol (DAG) of a fatty acid molecule (Arabolaza et al., 2008). The vector pIJE486 (Ylihonko et al., 1996) was used to carry the gene sco095 to Streptomyces strain.

**G011** is a mutant of GAL4111 and does not produce detectable quantities of bioactive metabolites. GAL4111 has been identified to be *Streptomyces roseosporus* based on identical patterns of DNA sequence, colony characteristics and ability to produce small quantities, some milligrams per litre of Daptomycin analogues. Colony morphology: On ISP4 agar G011 form spores with white/red pigment while in the reverse side of agar plate, the substrate mycelia is from reddish to brown and a thin clear zone around these red/brown colonies is visible indicating an amylase activity. In a rich agar, ISP2, G011 colonies are brown, slight sporulation with white/red pigment is seen. The substrate mycelia on ISP2 are slightly red with brownish aerial hyphae. In submerged cultivations it provides reddish pellet or dispersed mycelia. The strain forms spores also in submerged cultivations e.g. in TSB and in E05. The strain has increased cell mass as compared to its parent strain. The accumulation of TAGs was 1 g/l. GAL 4111 corresponds to *S*. *roseosporus* strain NRRL 11379. The sequence identity of GAL4111 to the NRRL strain was > 98% when DNA fragment of 1431 bp of 16S ribosomal RNA gene GI:195979310 were compared.

G009 is a *Streptomyces albus* GAL1001 strain carrying plasmid pIJEsco0958 (descibed above). It does not produce detectable quantities of bioactive metabolites. Colony morphology: On ISP4 agar G009 form spores with white pigment while in the reverse side of agar plate; the colonies are brown surrounding by a thin bright zone. In a rich agar, ISP2, G009 colonies form yellow substrate mycelia and yellowish aerial hyphae. Spores of white pigment are slightly visible. Typical morphology in submerged culture is pellet or dispersed mycelia. The strain forms spores also in submerged cultivations e.g. in TSB and in E05. The relevant characters according to this disclosure are increased cell mass as compared to the parent strain and accumulation of TAGs in a variety of cultivation condition up to 30 g/l. The characters of the strain are stable in passages up to 20 times tested so far. GAL 1001 corresponds to *S*. *albus* strain DSM 40313. The sequence identity of GAL1001 to the DSM strain was > 98% when DNA fragment of 1499bp of 16S ribosomal RNA gene GI:219878476 were compared.

### DEPOSITED MICRO-ORGANISMS

Streptomyces sp. G011 strain was deposited under Budapest Treaty on 15 December 2009 to DSMZ-Deutsche Sammlung fur Mikroorganismen und Zellkulturen GmbH, and it received the Accession number DSM 23182. Biological material was deposited under accession number DSM 23182 at DSMZ-Deutsche Sammlung für Mikroorganismen und Zellkulturen GmbH by Galilaeus Oy of Kairiskulmantie 10, 20781 Kaarina, Finland. Galilaeus Oy authorises the applicant to refer to the aforesaid biological deposit in the present application and applications /patents claiming priority from this application, and gives his unreserved and irrevocable consent to the deposited material being made available to the public in accordance with the applicable national laws.

Various embodiments of the invention are described below with the aid of the following numbered paragraphs 1-23:
1. A process for treating palm oil production residue(s), wherein the process comprises one or more aerobic treatment steps, which comprise(s)
   - cultivating lipid producing heterotrophic and/or mixotrophic microorganisms in a medium comprising palm oil production residue(s) as carbon and as nutrient source(s) under aerobic cultivation conditions;
   - separating the solids fraction from the liquid fraction; and
   - recovering lipids from the solids fraction.
2. The process according to paragraph 1, wherein lipid producing heterotrophic and/or mixotrophic microorganisms are cultivated under aerobic and aerated cultivation conditions.
3. The process according to paragraph 1 or 2, wherein the palm oil production residue(s) comprise(s) palm oil mill effluent (POME) and optionally additional lignocellulosic palm oil production residue(s) or fractions thereof.
4. The process according to paragraph 3, wherein the additional lignocellulosic palm oil production residue(s) comprise(s) empty fruit bunches (EFB), palm fruit fibre, such as mesocarp fibre; palm kernel shell; palm kernel cake; POME solids; oil palm wood, such as trunks, branches, roots or fractions thereof; or oil palm fronds, such as leaves; or fractions, hydrolysates or combinations thereof.
5. The process according to any one of paragraphs 1 to 4, wherein the cultivation medium comprise(s) POME and added lignocellulosic palm oil production residue(s) or hydrolysates thereof 0.1 - 400 g as dry weight per liter cultivation medium, preferably 1 - 300 g/l, more preferably 2 to 200 g/l.
6. The process according to any one of paragraphs 1 to 5, wherein the microorganisms belong to yeasts, moulds, bacteria or heterotrophic microalgae or any mixed population thereof.
7. The process according to paragraph 6, wherein the origin of the mixed population is palm oil mill effluent (POME).
8. The process according to any one of paragraphs 1 to 7, wherein the process comprises one or more aerobic, preferably aerated cultivation steps, wherein from the first aerobic, preferably aerated cultivation is introduced sludge into the second aerobic, preferably aerated cultivation, both cultivations comprising lipid accumulating heterotrophic and/or mixotrophic microorganisms.
9. Process according to paragraph 8, wherein the second aerobic, preferably aerated treatment is fed with lignocellulosic palm oil production residues or fractions or hydrolysates thereof.
10. The process according to any one of paragraphs 1 to 9, wherein the microorganisms comprises a mixed population with autotrophic microorganisms or autotrophic microalgae.
11. The process according to any one of paragraphs 1 to 10, wherein the microorganisms comprise actinomycetes, preferably *Streptomyces,* more preferably *Streptomyces roseosporus, Streptomyces albus or Streptomyces milbemycinius.*
12. The process according to any one of paragraphs 1 to 11, wherein the cultivation medium comprises an additional carbon source, such as purified sugar(s) or crude sugar(s).
13. The process according to any one of paragraphs 1 to 12, wherein the cultivation medium comprises cell waste or sludge from waste water treatment or any mixtures thereof.
14. The process according to any one of paragraphs 1 to 13, wherein the treatment is carried out in an aerated pond.
15. The process according to paragraph 1 to 14, wherein the treatment is carried out in an activated sludge process.
16. The process according to any one of paragraphs 1 to 13, wherein the treatment is carried out in a closed bioreactor.
17. The process according to any one of paragraphs 1 to 16, wherein the process comprises one or more anaerobic treatment steps, from which the effluent is introduced to aerobic, preferably aerated heterotrophic and/or mixotrophic cultivation with lipid accumulating microorganisms.
18. The process according to any one of paragraphs 1 to 17, wherein the process comprises one or more aerobic, preferably aerated treatment steps with heterotrophic and/or mixotrophic organisms and from which the effluent is introduced to anaerobic treatment.
19. The process according to any one of paragraphs 1 to 18, wherein the process comprises one or more anaerobic treatment steps, from which the effluent is introduced to aerobic auto- and/or mixotrophic cultivation with lipid accumulating microorganisms.
20. The process according to paragraphs 18 and 19 where the anaerobic process comprises an anaerobic digestion process, a hydrogen production process and/or an acidogenesis process.
21. The process according to any one of paragraphs 1 to 20, wherein the effluent from heterotrophic aerobic, preferably aerated cultivation with lipid accumulating microorganisms is introduced into auto- and/or mixotrophic lipid production with microalgae and/or cyanobacteria.
22. The process according to any one of paragraphs 1 to 20, wherein the effluent from lipid production is recirculated to the palm oil production process, used in irrigation, introduced to anaerobic digestor, introduced to algae pond, introduced to aerobic waste water treatment or is released to the environment optionally after further treatment steps.
23. Use of the lipids produced according to the process according to any one of paragraphs 1- 22 as biofuel, as a component of biofuel or as a starting material for biofuel production or as lubricant, as a component of lubricant or as a starting material for lubricant production.
24. The use according to paragraph 23, wherein the biofuel is biodiesel or renewable diesel, gasoline and/or jet fuel.

The following non-limiting examples illustrate the invention.

### Examples

### Characteristics of POME

The characteristics of POME used in the experiments are specified in Table 1:

**Table 1. Characteristics of palm oil mill effluent used in the experiments**

| **Characteristic** | **Value** |
|---|---|
| pH | 3.6 |
| DO | 8.8 mg/L |
| BOD | 53-57 g/L |
| CODₛ | 31 g/L |
| CODₜₒₜ | 84 g/L |
| VSS | 40 g/L |
| Ntot | 1.1 g/L |
| NH₄-N | 45 mg/L |
| NO₂⁻ | ? |
| NO₃⁻ | ≈0 |
| Pₜₒₜ | 0.11 g/L |
| PO₄³⁻ | 260 mg/L |

The following methods for analysis were used in the experiments involved in Examples if not otherwise stated in the text.

### Methods:

**Detection of packed mycelia volume (PMV):** Packed mycelia volume was measured by taking 10 ml sample of culture broth to a Falcon tube and centrifuged for 10 minutes at 4000 rpm. After centrifugation the supernatant was discarded and volume of packed cell mass was detected. The PMV was given as % value of the culture broth.

**Visual analysis of culture:** The bacterial cultures were visually analysed by separated colonies on agar plate, by studying liquid cultures and by microscopic analysis.

**TLC analysis:** 5 µl of Chloroform extract was pipetted on TLC plate (Silica gel 60 F254 Merck 1.05729) and run in hexane:diethyl ether:acetic acid (80:20:2). Lipids were coloured with iodine after the run. Cholesterol (C), cholesterol octadecanat (COD), decanoic acid, glyceryl trimyristate (GTM) and L-α-phospatidylethanolamine were used as standards on TLC. Lipids were visually detected on TLC-plates as yellow (iodine) spots. **Analysis of TLC spots:** Lipids were visually detected on TLC-plates as yellow spots. More clearly iodine coloured spots were seen in UV light. Sample spots were compared with GTM standard curve used at each TLC plate to estimate the quantities in g/l. The standard curve was prepared using concentration scale from 1 g/l up to 40 g/l and the titre of lipids in the culture broth was measured against the standard curve. Estimation was given based on the intensity of the spots as compared to GTM standard concentrations. **Sample extraction:** The whole culture broth (solid stage cultivations) was extracted with an equal volume of chloroform-methanol (Clf-MeOH; 2:1). Tubes were incubated in a shaker at room temperature (RT) for 1 hour. After centrifugation sample was taken from the extract and analysed by TLC and/or by GC.

**Detection of dry extraction weight:** Dry extract weight was determined by drying 1 ml of extract at RT for one day in air blow after which mass was determined by weighing. The mass was given as grams per 1 litre of the culture broth.

### Example 1. Use of palm oil production residues in the cultivation of Streptomyces

The capability of *Streptomyces albus and S.* roseosporus strains G009 and G012 were tested to be able to use Empty fruit bunches (EFB) and Palm oil mill effluent (POME) as sole raw material for growth and for lipid accumulation.

Typical cultivation conditions:
Temperature: 28 °C
Agitation: 200-330 rpm (3/4-1 inch throw)
Inoculum form: vegetative mycelia, substrate mycelia, spores
Transferring rate: 1-10 %
Cultivation volume: flask scale (about 100 - 1000 ml)
Seeding stages performed: Inoculation from spore stock to media
Standard medium in use: POME and EFB alone; EFB 10-50 g/I, POME 50-100 % (POME to be added up to 1 L is given here as 100 %)

Characteristics of the *Streptomyces* culture:
Time for exponential growth: 12-48 h
Time to accumulate lipids: 30-144 h
The mass of chloroform extract: > 4 g/l of culture broth
The TAG fraction of lipids extracted: 20-80 vol-%
Increase of TAGs: 1 g/l from the beginning of cultivaiton (time for highest value 3 days).

In conclusion, it was shown that Streptomyces strains G009 and G012 strains were able to grow and produce lipids on sole POME and EFB material.

### Example 2. Use of palm oil waste combined with nitrogen source in cultivation of Streptomyces

The strains *Streptomyces albus* G009 and *Streptomyces roseosporus* G012 were cultivated under conditions as described below. A combination of EFB and POME materials were used as C-source in complex media.

Typical cultivation conditions:
Temperature: 22-36 °C
Agitation: 200-330 rpm
Inoculum form: vegetative mycelia, substrate mycelia, spores
Transferring rate: 0.5 - 50 %

Culture media:
1) a combination of EFB and POME; EFB 20 g/l POME (POME 100 %),
2) Culture medium E05, in which EFB replaces starch and POME replaces dextrose partly or completely.

E05 contains the following ingredients: 20.0 g/l of dextrose, 20.0 g/l of starch, 5.0 g/l of farmamedia, 2.5 g/l of yeast extract, 1.0 g/l of MgSO₄ 7 H₂O, 1.0 g/l of KH₂PO₄, 3.0 g/l of CaCO₃, 3.0 g/l of NaCl.

Characteristics of the cultivation:
Time for exponential growth: 12-64 h
Time to accumulate lipids: 24-96 h

The mass of chloroform extract: > 6 g/l of culture broth

The TAG fraction of lipids extracted: 30-80 wt-%Increase of TAGs from the beginning of cultivation: 5 g/l (time for highest value 3 days)

In conclusion it was shown that POME and EFB are suitable carbon sources for lipid production by *Streptomyces.* This example indicates that POME and lignocellulosic palm oil production residues are suitable raw materials for lipid production by *Streptomyces.*

### Example 3. Content of the cultivation broth: use of palm oil residues and cell waste to enhance the accumulation of lipids

To enhance the maximum growth and accumulation of TAGs in economical, low-cost, cultivation conditions, EFB and POME were used as carbon source and *Streptomyces* cell waste (extracted cell mass from previous fermentations) and/or corn steep liquor (CSL) were used as nutrient sources in cultivation media.

Characteristics of the Streptomyces cultivationTypical cultivation conditions:
Temperature: 22-36 °C
Agitation: 100-330 rpm
Inoculum form: vegetative mycelia, substrate mycelia, spores
Transferring rate: 1 - 50 %
Cultivation media: a combination of EFB and POME; EFB 20 g/l, POME as cultivation liquid (added up to make 1 L volume , called POME 100 %), with additional cell waste and/or CSL; 5-20 g/l each

Characteristics of the Streptomyces cultivation:
Time for exponential growth: 12-72 h
Time to accumulate lipids: 24-144 h
The mass of chloroform extract: > 10 g/l of culture broth
The TAG fraction of lipids extracted: 40-90 wt-%
Increase of TAGs: 15 g/l (time for highest value in 6 days) from the beginning of cultivation, total amount of TAGs 30 g/l
PMV: >28 %.

The example indicates that addition of cell waste and/or CSL to the cultivation medium including POME and EFB increased the lipid production by Streptomyces bacteria. No additional minerals were required to lipid production by Streptomyces from POME and EFB.

### Example 4. Media with POME, EFB and cell waste for enhanced lipid production

Streptomyces strain G009 was cultivated in the following broth composition EFB 20 g/I POME as cultivation liquid (added up to make 1 L volume), cell waste 20 g/I and ammonium sulphite 0.5 g/I.

Strain G009 was cultivated under the following conditions:
Temperature: 30 °C
Agitation: 300 rpm
Inoculum form: spores

Characters of the Streptomyces culture:
The mass of chloroform extract of 6 days: 30 g/l of culture broth
The TAG fraction of lipids extracted: 67 w-%
Increase of TAGs: 15 g/l (from 15 g/l (0 value) to 30 g/ in 6 day cultivation)

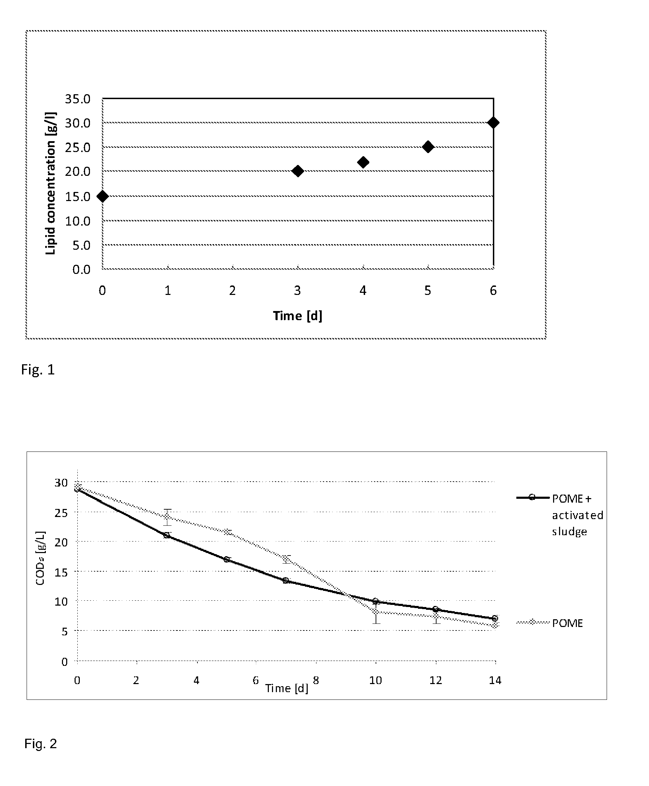

The cultivcation was performed for 6 days. Lipid content increased during the cultivationand after 6 days lipid concentration in cultivation broth of 30 g/L was obtained (Figure 1). Also good growth was observed and PMV values varied between 17 and 23 during cultivation.

Increase in the amount of cell waste and addition of ammonium sulphite gave robustness to the growth and accumulation of TAG. In conclusion, it was shown that supplementation of POME and EFB in combination with cell waste, such as Streptomyces cell waste, was beneficial to accumulation of lipids.

### Example 5. Batch fermentation of the strain G009

Strain G009 was cultivated in fermentation volume of 1.5 litre in a fermentor. The following procedure gives detailed information of the easily repeatable fermentation process. The following data was derived from cultivations of G009 in the each non-hydrolysed (depolymerized) waste; EFB + POME + cell waste. *Streptomyces* cells from previous fermentations were harvested by filtration and extracted by the equal volume of chloroform (1:1). The remaining oil-extracted cell residue was further dried to remove chloroform residuals at 60°C for 10 min to be supplied to the fermentation broth.

Strain G009 was cultivated under controlled fermentation conditions in the following range:
Cultivation medium: EFB 20 g/l POME as cultivation liquid (added up to make 1 L volume), with cell waste 20 g/L.
Inoculation: spores or a plug of agar plate culture
Transferring rate: 0.5 - 10 %
Seeding steps: 0-2
Temperature: 28 °C
Aeration: 0.5-1 vvm
Stirring: 100-280 rpm
Back pressure: 0.5 bar
pH after sterilization: 7
Cycle time: 144 hours

Characteristics of the *Streptomyces* cultivation:
Time for exponential growth: 20-140 h
Time to accumulate lipids: 32-144 h
The mass of chloroform extract: > 12 g/l of culture broth
The TAG fraction of chloroform extract: 50-60 w-%
The range of PMV %: 10-50
Increase of TAGs: 13 g/I (time for highest value 6 days)

The example shows that batch cultivation in fermentor can be used in lipid production by *Streptomyces* from palm oil production residues.

### Example 6. Oil accumulation by mixed cultures during treatment of POME

Oil accumulation by mixed culture microorganisms during treatment of POME was tested in 250 mL Erlenmeyer bottles. Two replicate cultures with 10% (v/v) of activated sludge from a municipal wastewater treatment plant (Viinikanlahti, Tampere) and 90% (v/v) POME and two replicate bottles with no additional inoculum (Milli-Q water was used to compensate the inoculum volume) and 90% (v/v) POME were incubated at 27 °C on orbital shakers at 300 rpm for 14 days. Medium pH was adjusted to 6.0 in the beginning of the incubation, but was not adjusted during the experiment.

The removal of soluble chemical oxygen demand (CODₛ) during treatment of POME with mixed cultures from municipal waste water treatment plant and inoculum included in POME is shown in Figure 2.

CODₛ in POME was in the beginning of the treatment 28-29 g/L and decreased to 5-7 g/I after 16 days of incubation (Figure 2). CODₛ consumption was very similar in bottles with added activated sludge inoculum and in bottles with no added inoculum, including only the microorganisms present in POME.

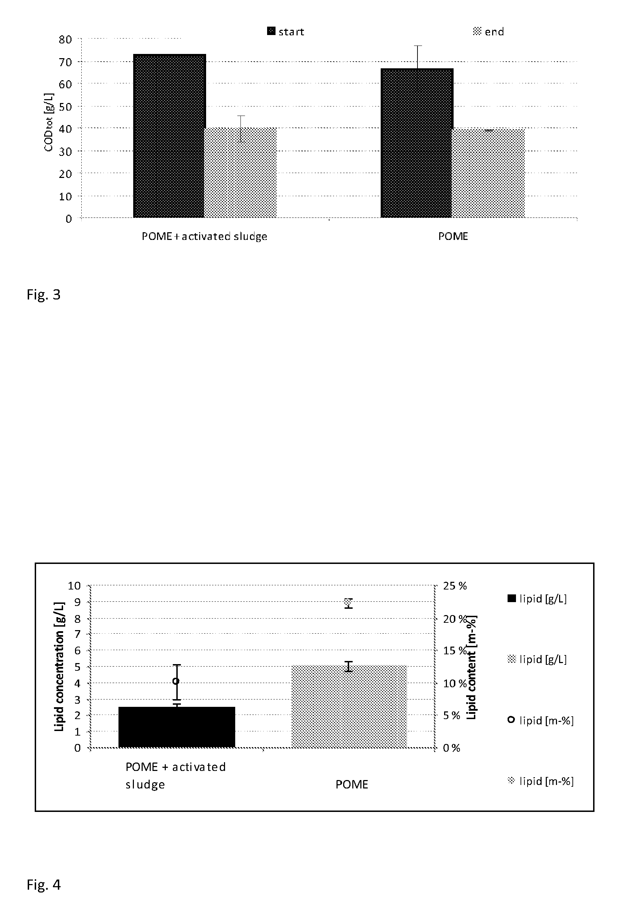

The removal of total chemical oxygen demand (CODₜₒₜ) during treatment of POME with mixed cultures from municipal waste water treatment plant and with no inoculum, including only the microorganisms included in POME is shown in Figure 3.

CODₜₒₜ values at the beginning were between 60 and 70 g/l and at the end (after 15d of treatment) between 30 and 40 g/l (Figure 3). Also total chemical (CODₜₒₜ) removal was similar in bottles with added activated sludge inoculum and in bottles with no added inoculum, including only the microorganisms present in POME.

The concentration of lipids in cultivation medium and lipid content in biomass during treatment of POME with mixed cultures from municipal waste water treatment plant and with no inoculums POME with mixed cultures from municipal waste water treatment plant and with no inoculum, including only the microorganisms included in POME is shown in Figure 4.

Lipid concentration in cultivation medium and lipid content in biomass was higher in the bottles with no inoculum including microorganisms only from POME compared to activated sludge inoculum (Figure 4).

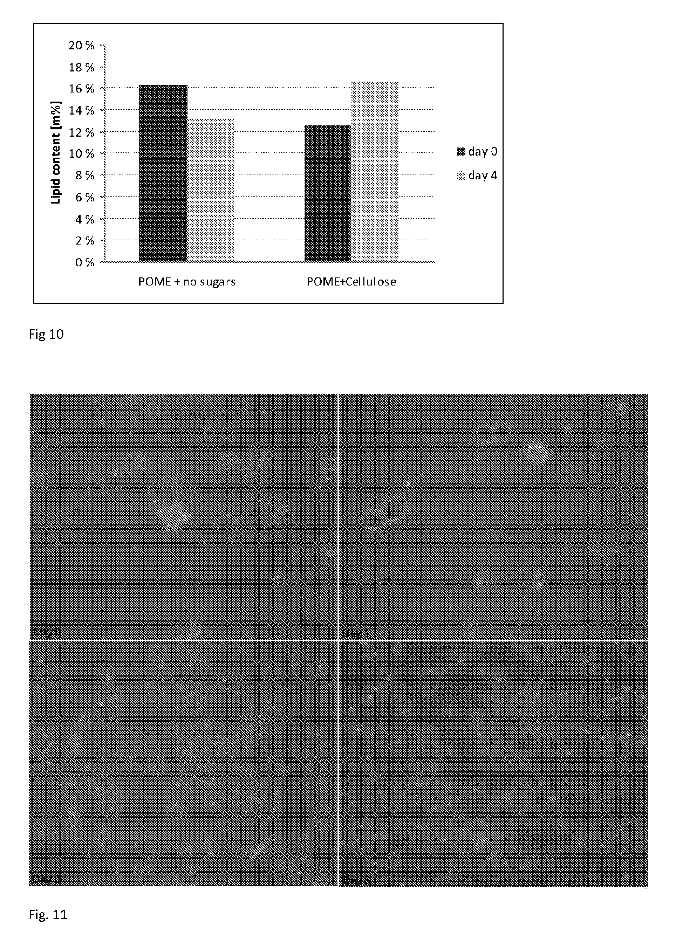

Examination with phase contrast microscopy showed microorganisms of POME to be able to accumulate lipids into the cells during 3 day incubation as shown in Figure 11. Photomicrographs were taken from incubation of POME and milli-Q water in ratio 90:10 (v/v). Incubation was conducted in 500 mL Erlenmeyer bottles with initial liquid volume of 150 mL at 27 °C and 250 rpm.The example indicates that microorganisms capable of accumulating lipids exists in POME and can be enriched.

The example shows similar COD removal in cultures with activated sludge and POME microorganisms and just microorganisms included in POME. The example also shows lipid accumulation by mixed culture microorganisms during treatment of POME.

### Example 7. Effect of addition of carbon source on oil accumulation by mixed cultures during treatment of POME

The effect of carbon addition on lipid production during treatment of POME was studied. Inoculum was municipal activated sludge (secondary sludge) from waste water treatment facility, (Viinikanlahti, Tampere). Culture pH was adjusted to 6 with 10 M NaOH and 1 M H₂SO₄ at the beginning of the experiment and after each sampling. Cultivations were done in duplicate 1000 mL Erlenmeyer flasks at 27 °C on orbital shakers at 250 rpm. Bottles contained 90% POME (v/v) and 10% (v/v) activated sludge enrichment. 2 g/l of glucose was added to one of the bottles during cultivation on days 4, 7, 10 and 14.

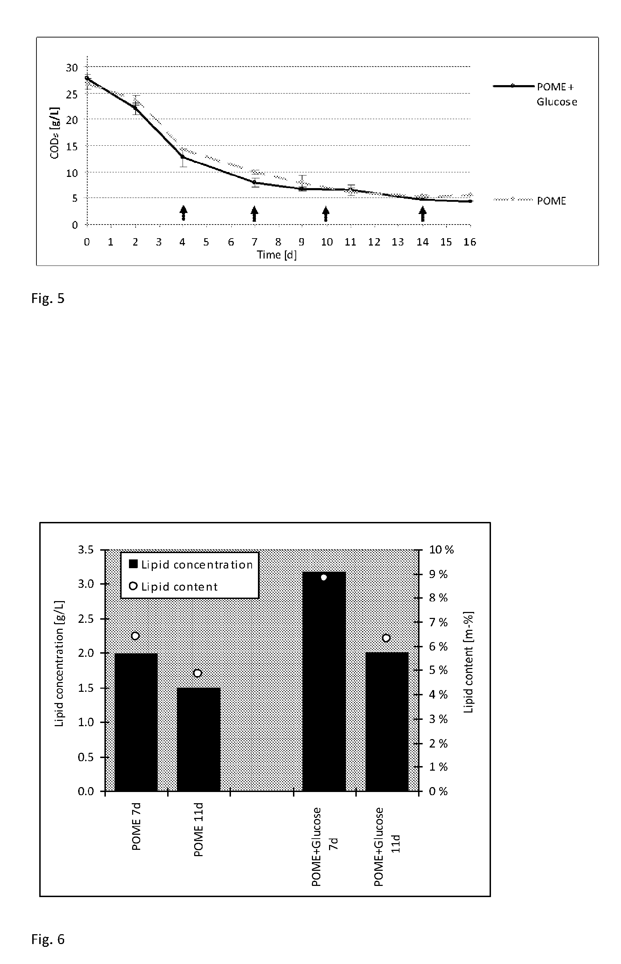

The initial chemical oxygen demand in POME in the experiment was about 26 to 28 g/l. During the treatment, despite of adding carbon to cultivation bottles, the dissolved chemical oxygen demand decreased to about 5-7 g/L during treatment in about 15 days (Figure 5).

The concentration of lipids in cultivation medium and lipid content in biomass during treatment of POME with mixed cultures with and without addition of glucose after 7 and 11 days of cultivation is shown in Figure 6.

The results show that the amounts of lipids in cultivation medium and in biomass (lipid content) was higher in bottle where carbon was added. The lipid content in biomass was lower after 11 d than after 7d. Thus, the lipids can degrade during cultivation and the optimal time for collection of biomass need to be determined in each case.

The results indicate that the lipid accumulation during treatment of POME can be increased by addition of carbon, such as sugars, in the cultivation medium.

### Example 8. Lipid accumulation by mixed cultures on POME and lignocellulosic hydrolysates

Effect of external carbon source, glucose and xylose mixture representing lignocellulosic hydrolysates, addition on treatment of POME and accumulation of lipids was tested with an activated sludge enrichment inoculum in 1000 mL Erlenmeyer flasks. One bottle contained 90% POME (v/v) and 10% (v/v) activated sludge enrichment and another bottle contained 90% POME (v/v) and 2 g/l glucose/xylose-mixture in ratio of 2:1 were incubated at 27 °C on an orbital shaker at 250 rpm for 8 days. Medium pH was adjusted to 6.0 in the beginning of the experiment.

Activated sludge enrichment culture had been enriched from activated sludge from a municipal wastewater treatment plant (Viinikanlahti, Tampere) with POME at 27 °C and 250 rpm by transferring 10% (v/v) culture from previous batch to fresh POME once a week.

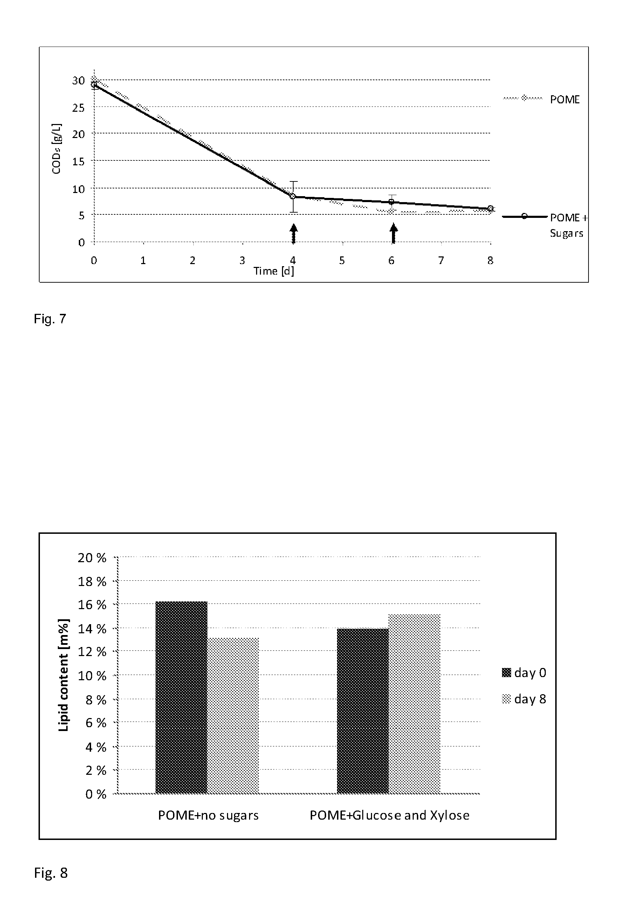

Figure 7 shows chemical oxygen demand (CODₛ) removal during treatment of POME with mixed cultures with and without additions of sugars during treatment. Consumption of CODₛ compounds was similar in cultures with and without external carbon addition. The residual CODₛ in bottle with sugar addition was higher than in bottle without added carbon.

The lipid content in biomass during treatment of POME with mixed cultures with and without addition of sugars, representing lignocellulose hydrolysate composition, is shown in Figure 8.

In bottle with POME and no external carbon addition biomass lipid content slightly decreased between cultivation days 0 and 8, but in the bottle with POME and sugar addition, representing lignocellulosic hydrolysate composition, lipid content increased (Figure 8).

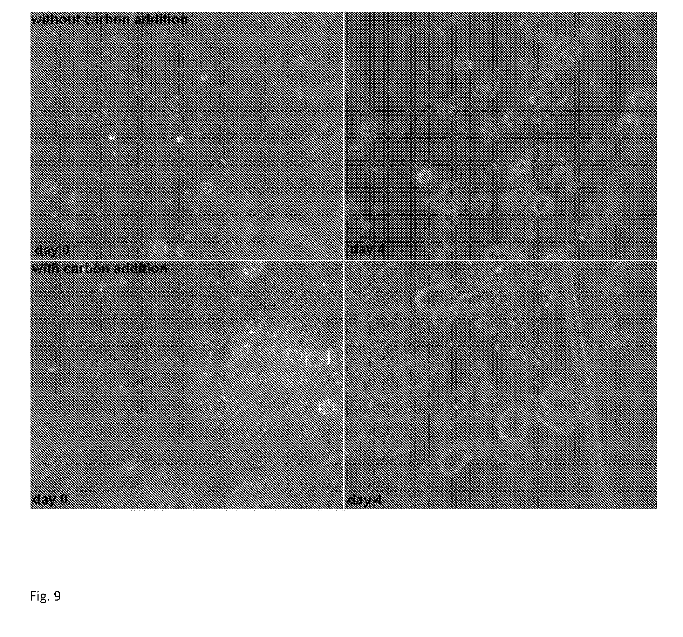

Microscopic analysis of the cultures with sugar addition, representing lignocellulosic hydrolysate composition, demonstrated lipid inclusions in eukaryotic cells after 5 days of incubation where as after 1 day smaller cells without lipid inclusions were detected (Figure 9). More cells with lipid inclusions were seen after five days in cell culture grown on POME with sugar addition than in that without addition of sugars.

The results show that lipid accumulation during treatment of POME by mixed culture microorganisms can be increased by addition of external carbon source in the cultivation medium. In addition it is shows that hydrolysates of lignocellulosic materials, such as hydrolysates of lignocellulosic residues from palm oil production, can be used as the source of this external carbon.

### Example 9. Lipid accumulation by mixed cultures on POME and cellulose

Effect of external carbon source, fibrous cellulose representing a fraction of lignocellulose addition on treatment of POME and accumulation of lipids was tested with an activated sludge enrichment inoculum in 1000 mL Erlenmeyer flasks. Two bottles contained 90% POME (v/v) and 10% (v/v) activated sludge enrichment and another two bottles contained 90% POME (v/v) and 2 g/L fibrous cellulose (fiber, long, from Sigma) were incubated at 27 °C on an orbital shaker at 250 rpm for 4 days. Medium pH was adjusted to 6.0 in the beginning of the experiment.

The lipid content in biomass during treatment of POME with mixed cultures with and without addition fibrous cellulose is shown in Figure 10. In bottle with POME and no external carbon addition lipid content in biomass decreased between cultivation days 0 and 4, but in the bottle with POME and cellulose addition lipid content in biomass increased during cultivation (Figure 10).

The results show that lipid accumulation during treatment of POME by mixed culture microorganisms can be increased by addition of external carbon source, such as polymeric carbon from lignocellulose, in the cultivation medium. The polymeric carbon sources can be obtained e.g. from lignocellulosic palm oil production residues.

### References

Arabolaza, A, Rodriguez, E, Altabe, S, Alvarez, H and Gramajo, H (2008) Multiple pathways for triacylglyserol biosynthesis in Streptomyces coelicolor. Appl Env Microb 79: 2573-2582.
Gräfe, U., Reinhardt, G., Hänel, F., Schade, W., Gumpert, J. (1985). Occurrence or squalene and dehydrosqualene in streptomycets. J. Basic Microbiol. 25(8):503-507
Gunstone, F.D., Harwood, J.L., Dijkstra, A.J. (eds) (2007) The lipid handbook, 3rd edn. CRC Press, Boca Raton.
Lam MK, Lee KT. 2011. Renewable and sustainable bioenergies production from palm oil mill effluent (POME): Win-win strategies toward better environmental protection. Biotechnology Advances 29:124-141.
Olukoshi, E.R., Packter, N.M. (1994). Importance of stored triacylglycerols in Streptomyces: possible carbon source for antibiotics. Microbiology 140:931-943.
Oswal N, Sarma PM, Zinjarde SS, Pant A. 2002. Palm oil mill effluent treatment by a tropical marine yeast. Bioresource Technology 85:35-37.
Ratledge, C., Cohen, Z. 2008. Microbial and algal oils: Do they have a future for biodiesel or as commodity oils. Lipid Technology 20:155-160.
Vishnupriya, B.Sundaramoorthi,C..Kalaivani, M., Selvam, K.: International Journal of ChemTech Research, Vol.2, No.3, pp 1380-1383, July-Sept 2010, Production of lipase from Streptomyces griseus and evaluation of Bioparameters.
Wu T.Y., Mohammad A.W., Jahim J.M., Anuar, N. 2009. A holistic approach to managing palm oil mill effluent (POME): Biotechnological advances in the sustainable reuse of POME. Biotechnology Advances 27 (2009) 40-52.
Ylihonko, K, Tuikkanen, J, Jussila, S, Cong, L and Mäntsälä, P (1996) A gene cluster involved in nogalamycin biosynthesis from Streptomyces nogalater: sequence analysis and complementation of early-block mutations in the anthracycline pathway. Mol Gen Genet 251:113-120.

## Claims

1. A process for treating palm oil production residue(s), wherein the process comprises one or more aerobic treatment steps, which comprise(s)
- cultivating lipid producing heterotrophic and/or mixotrophic microorganisms in a medium comprising palm oil production residue(s) as carbon and nutrient source(s) under aerobic cultivation conditions;
- separating the solids fraction from the liquid fraction; and
- recovering lipids from the solids fraction.

2. The process according to claim 1, wherein lipid producing heterotrophic and/or mixotrophic microorganisms are cultivated under aerobic and aerated cultivation conditions.

3. The process according to claim 1 or 2, wherein the palm oil production residue(s) comprise(s) palm oil mill effluent (POME) and optionally comprise additional lignocellulosic palm oil production residue(s), preferably empty fruit bunches (EFB), palm fruit fibre, palm kernel shell, palm kernel cake, POME solids, oil palm wood or oil palm fronds or fractions, hydrolysates or combinations thereof.

4. The process according to any one of claims 1 to 3, wherein the cultivation medium comprise(s) POME and added lignocellulosic palm oil production residue(s) or hydrolysates thereof in an amount of 0.1 - 400 g as dry weight per liter cultivation medium, preferably 1 - 300 g/l, more preferably 2 to 200 g/l.

5. The process according to any one of claims 1 to 4, wherein the microorganisms comprise yeasts, moulds, bacteria or heterotrophic microalgae or any mixed population thereof.

6. The process according to any one of claims 1 to 5, wherein the process comprises one or more aerobic, preferably aerated cultivation steps, wherein from the first aerobic, preferably aerated cultivation step is introduced sludge into the second aerobic, preferably aerated cultivation step, both cultivations comprising lipid accumulating heterotrophic and/or mixotrophic microorganisms; and wherein optionally the second aerobic, preferably aerated step is fed with lignocellulosic palm oil production residues or hydrolysates thereof.

7. The process according to any one of claims 1 to 6, wherein the microorganisms comprise a mixed population with autotrophic microorganisms or autotrophic microalgae.

8. The process according to any one of claims 1 to 7, wherein the microorganisms comprise actinomycetes, preferably *Streptomyces,* more preferably *Streptomyces roseosporus, Streptomyces albus or Streptomyces milbemycinius.*

9. The process according to any one of claims 1 to 8, wherein the cultivation medium comprises an additional carbon source, such as purified sugar(s) or crude sugar(s).

10. The process according to any one of claims 1 to 9, wherein the cultivation medium comprises cell waste or sludge from waste water treatment or any mixtures thereof.

11. The process according to any one of claims 1 to 10, wherein the treatment is carried out in an aerated pond, in an activated sludge process or in a closed bioreactor.

12. The process according to any one of claims 1 to 11, wherein the process comprises one or more anaerobic treatment steps, from which the effluent is introduced to aerobic,preferably aerated heterotrophic and/or mixotrophic cultivation with lipid accumulating microorganisms.

13. The process according to any one of claims 1 to 12, wherein the process comprises one or more aerobic, preferably aerated treatment steps with heterotrophic and/or mixotrophic organisms and from which the effluent is introduced to anaerobic treatment.

14. The process according to any one of claims 1 to 13, wherein the process comprises one or more anaerobic treatment steps, from which the effluent is introduced to aerobic auto- and/or mixotrophic cultivation with lipid accumulating microorganisms.

15. The process according to any one of claims 12 to 14 where the anaerobic process comprises an anaerobic digestion process, a hydrogen production process and/or an acidogenesis process.

16. The process according to any one of claims 1 to 15, wherein the effluent from heterotrophic aerobic, preferably aerated cultivation with lipid accumulating microorganisms is introduced into auto- and/or mixotrophic lipid production with microalgae and/or cyanobacteria.

17. Use of the lipids produced according to the process according to any one of claims 1-16 as biofuel, as a component of biofuel or as a starting material for biofuel production or as lubricant, as a component of lubricant or as a starting material for lubricant production

18. The use according to claim 17, wherein the biofuel is biodiesel or renewable diesel, gasoline and/or jet fuel.
